# EUROPEAN PATENT SPECIFICATION

(11) **EP 1 474 689 B1**
(45) Date of publication and mention of the grant of the patent: **12.09.2012**
(21) Application number: 03708103.1
(22) Date of filing: 17.02.2003
(51) Int. Cl.: G01N 33/564, G01N 33/68

(54) **METHOD OF DIAGNOSIS OF INFLAMMATORY DISEASES USING CALGRANULIN C**
VERFAHREN ZUR DIAGNOSE VON ENTZÜNDUNGSERKRANKUNGEN UNTER VERWENDUNG VON CALGRANULIN C
METHODE DE DIAGNOSTIC DE MALADIES INFLAMMATOIRES A L'AIDE DE LA CALGRANULINE C

(30) Priority: 15.02.2002 US 77600
(43) Date of publication of application: 10.11.2004
(73) Proprietor: Sorg, Clemens, 48161 Münster (DE); Roth, Johannes, 48153 Münster (DE)
(72) Inventor: Sorg, Clemens, 48161 Münster (DE); Roth, Johannes, 48153 Münster (DE)
(74) Representative: Koch, Andreas
(86) International application number: PCT/EP2003/001575
(87) International publication number: WO 2003/069341

(56) References cited:
- EP-A- 0 731 166
- WO-A-00/20621
- WO-A-01/86002
- WO-A-02/48310
- WO-A-03/060465
- YANG Z. ET AL.: "Proinflammatory properties of the human S100 protein S100A12." J. LEUKOC. BIOL., vol. 69, no. 6, June 2001 (2001-06), pages 986-994, XP009019576
- FOELL D. ET AL.: "S100A12 (EN-RAGE) in monitoring Kawasaki disease." LANCET, vol. 361, 2003, pages 1270-1272, XP004420127
- ROTH J. ET AL.: "Phagocyte-specific S100 proteins: a novel group of proinflammatory molecules." TREIMM, vol. 24, no. 4, April 2003 (2003-04), pages 155-158, XP004419757
- FOELL D. ET AL.: "Neutrophil derived human S100A12 (EN-RAGE) is strongly expressed during chronic active inflammatory bowel disease", GUT, vol. 52, no. 6, June 2003 (2003-06), pages 847-853,
- FOELL D. ET AL.: "Phagocyte-specific calcium-binding S100 proteins as clinical laboratory markers of inflammation", CLIN. CHIM. ACTA, vol. 344, no. 1-2, June 2004 (2004-06), pages 37-51,
- FOELL D. ET AL.: "S100 proteins expressed in phagocytes: a novel group of damage-associated molecular pattern molecules", J. LEUK. BIOL., vol. 81, no. 1, January 2007 (2007-01), pages 28-37,

SEQUENCE LISTING
<110> Clemens Sorg and Johannes Roth
<120> Method for diagnosis of inflammatory diseases using Calgranulin C
<130> S30274PCT
<160> 2
<170> PatentIn version 3.1
<210> 1
   <211> 466
   <212> DNA
   <213> homo sapiens
<400> 1
   accactgctg gctttttgct gtagctccac attcctgtgc attgaggggt taacattagg     60
   ctgggaagat gacaaaactt gaagagcatc tggagggaat tgtcaatatc ttccaccaat    120
   actcagttcg gaaggggcat tttgacaccc tctctaaggg tgagctgaag cagctgctta    180
   caaaggagct tgcaaacacc atcaagaata tcaaagataa agctgtcatt gatgaaatat    240
   tccaaggcct ggatgctaat caagatgaac aggtcgactt tcaagaattc atatccctgg    300
   tagccattgc gctgaaggct gcccattacc acacccacaa agagtaggta gctctctgaa    360
   ggctttttac ccagcaatgt cctcaatgag ggtcttttct ttccctcacc aaaacccagc    420
   cttgcccgtg gggagtaaga gttaataaac acactcacga aaagtt                   466
<210> 2
   <211> 92
   <212> PRT
   <213> homo sapiens
<400> 2
   Met Thr Lys Leu Glu Glu His Leu Glu Gly Ile Val Asn Ile Phe His
   1               5                   10                  15
   Gln Tyr Ser Val Arg Lys Gly His Phe Asp Thr Leu Ser Lys Gly Glu
               20                  25                  30
   Leu Lys Gln Leu Leu Thr Lys Glu Leu Ala Asn Thr Ile Lys Asn Ile
           35                  40                  45
   Lys Asp Lys Ala Val Ile Asp Glu Ile Phe Gln Gly Leu Asp Ala Asn
       50                  55                  60
   Gln Asp Glu Gln Val Asp Phe Gln Glu Phe Ile Ser Leu Val Ala Ile
   65                  70                  75                  80
   Ala Leu Lys Ala Ala His Tyr His Thr His Lys Glu
                   85                  90

## Description

### FIELD OF INVENTION

The present invention is directed to a method for diagnosing inflammatory diseases, particularly for diagnosing specific stages of inflammatory diseases and/or for determining the risk of relapse and/or for discriminating between diseases with similar symptoms based on the marker CALGRANULIN C.

### BACKGROUND OF THE INVENTION

A lot of diseases are characterized by symptoms of inflammation (inflammatory diseases). An indication is the presence of inflammatory cells such as neutrophils and macrophages at local sites of inflammation. The inflammatory state can also be systemic, i.e. proteins secreted by inflammatory cells become detectable in the blood serum.

In spite of different or very often unknown pathogenic background, the early symptoms of inflammatory diseases may be very similar; e.g. fever is a very common symptom of acute inflammatory diseases. Known causes for inflammatory diseases are autoimmune reactions, bacterial, viral or parasite infections, genetic disorders, allergies. In many cases, mixtures of these or other causes have been proposed, e.g. for the very common disease psoriasis, which is characterized by inflammation of the epidermis. In some cases of psoriasis patients, also the locomotive system may be affected resulting in psoriatic arthritis. Especially the joints are affected by strong inflammation in this disease, eventually resulting in stiffness. This disease is characteristic in presumably being caused by multiple factors such as genetic predisposition, psychological stress or irritation of the skin.

The different forms of chronic inflammatory arthritis comprise a heterogeneous group of clinically relevant disorders affecting general mesenchymal tissues. This is leading to severe destruction of joint tissue, resulting in cartilage and bone damage, and contributing to a large degree of disability among patients. Inflammation of the synovial tissue is a common feature of peripheral joint disease in rheumatoid arthritis. Synovial inflammation or "synovitis" is characterized by hyperplasia of the lining layer and cellular infiltration and hypervascularity of the sublining layer. In addition to T-lymphocytes, phagocytes have a crucial role in the pathogenesis of synovial inflammation by secretion of various pro-inflammatory cytokines and metalloproteinases (Bresnihan & Tak, 1999, Res Clin Rheumatol 13: 645-659). Acute exacerbations are characteristic for rheumatoid arthritis. Aetiology is largely unclear, but an autoimmune disease background is suggested.

In children, juvenile rheumatoid arthritis (JRA), also referred to as juvenile chronic arthritis (JCA) or juvenile idiopathic arthritis (JIA), is the most frequent rheumatic autoimmune disease. Children up to 16 years are affected. Among the group of different forms of JRA, systemic onset juvenile rheumatoid arthritis (SOJRA) or Still's disease is the most severe and dangerous form. SOJRA is characterized by a systemic inflammatory reaction which involves several organ systems, e.g. spleen, liver, lymph nodes, bone marrow and skin. During the further course of this disease patients develop a severe arthritis which often is refractory to anti-inflammatory therapy. The pathogenesis of this disorder is completely unknown. Patients with SOJRA show no characteristic immunological features at initial presentation but rather a general activation of their innate immune system, e.g. thrombocytosis, neutrophilia and activation of the complement system. This non-specific inflammatory pattern is responsible for the difficulties with regard to the early diagnosis, especially with regard to discrimination from bacterial infections. The fact that SOJRA resembles bacterial infections in early symptoms and that no reliable diagnosis marker exists, makes it in addition very difficult to choose the correct medication very early.

An exact regulation of treatment of the different forms of JRA by administration of anti-inflammatory substances can only be performed insufficiently to date. Pathogenesis of the different disease forms is largely unclear and hence, therapy cannot be directed to a specific target. Especially the endpoint of treatment represents a major problem in medication: about 50% of the JRA patients relapse after withdrawal of the treatment with methotrexat (MTX) (Ravelli et al., 1995, J Rheumatol 22: 1574). Several authors have therefore proposed to treat JRA patients with immunosuppressants for several years even after clinical remission. To date, no reliable parameters exist to determine residual inflammatory activity of rheumatoid arthritis diseases quickly and sensitively in order to exclude the risk of relapse. Common inflammatory parameters as C-reactive protein (CRP) or erythrocyte sedimentation rate (ESR) lack specificity and sensitivity (Giannini and Brewer, 1987, Clin Rheumatol 6:197). Internationally accepted scores to determine disease activity mostly rely on clinical criteria (Giannini and Brewer, *supra*). This inadequate surveillance of disease activity results in steady treatment of the patients with immunsuppressant resulting in severe side effects (Giannini and Cassidy, 1993, Drug Saf 9: 325).

Psoriatic arthritis is usually not as destructive as rheumatoid arthritis which may be due to less synovial macrophage infiltration with a subsequent lower production of pro-inflammatory cytokines (Veale et al., 1993, Arthritis Rheum 36: 893-900; Danning et al., 2000, Arthritis Rheum 43: 1244-1256). Nevertheless, neutrophils are frequently present in synovitis in psoriatic arthritis and generalised up-regulation of neutrophil migration and secretion of lysosomal enzymes have been reported in psoriatic arthritis patients (Biasi et al., 1998, Inflammation 22: 533-543; Mikulikova et al., 1984, Clin Rheumatol 3: 515-519; Sedgwick et al., 1980, J Invest Dermatol 74: 81-84). In addition, altered vascular growth and function probably due to peculiar endothelial activation seem to play a primary role for synovitis in psoriatic arthritis (Reece et al., 1999, Arthritis Rheum 42: 1481-1484; Fearon et al., 1999, Ann NY Acad Sci 878: 619-621). Th1-cytokines, monokines, and vascular endothelial growth factor (VEGF) are present in psoriatic arthritis synovium and have been suggested to promote angiogenesis in psoriatic skin lesions (Fraser et al., 1991, Arthritis-Rheum 44: 2024-2028; Lowe et al., 1995, Br J Dermatol 132: 497-505). However, synovial expression of cytokines in psoriatic arthritis has been poorly characterized. (Ritchlin et al., 1998, J Rheumatol 25: 1544-1552).

Kawasaki disease, on the other hand, is an acute disease associated with fever and with multiple organs being affected. It is by far the most common systemic vasculitis in childhood. Children under the age of 1 year and boys are at special risk for fatal disease due to coronary artery abnormalities. However, the aetiology is largely unknown, although evidence points to an autoimmune disease in which neutrophils and endothelial cells are affected. Vasculitis, in particular Kawasaki disease, is a necrotising process predominantly affecting small and medium sized arteries. The aetiology and pathogenesis of vasculitis, in particular Kawasaki dis-ease, remains unclear. It may be best characterized by a generalised stimulation of inflammatory responses, possibly due to superantigens. The identification of a reliable marker for the diagnosis of the disease state and the identification of patients with an increased risk of heart complication would be advantageous for the adequate treatment of the patients.

Cystic fibrosis (CF) is a disease caused by genetic alterations with being the most common inherited lethal disease among whites with an estimated incidence of 1:3,400 live births. CF. transmembrane conductance regulator (CFTR) mutations lead to defective Cl⁻ transport in respiratory epithelium resulting in diminished mucus clearance. The consequence is enhanced production of mucus, chronic airway inflammation, recurrent infections and impaired host defense mechanisms. Chronic airway inflammation is the primary cause of morbidity and mortality. Pulmonary infections with a variety of Gram-positive and -negative bacteria, including atypical strains of *Staphylococcus aureus* and *Pseudomonas aeruginosa*, account to a large number of complications. Neutrophilic inflammation occurs early in life and contributes to progressive tissue changes. Acute exacerbations are a common reason for hospitalisation and antibiotic therapy. Due to the high level of chronic inflammation, it is very difficult to diagnose acute inflammatory excacerbations due to e.g. acquired bacterial infections. In order to ensure adequate treatment of this severe disease (only 80% of the patients get 19 years old or more), early diagnosis is a prerequisite.

One of the major problems lies in the diagnosis of acute exacerbations in patients suffering from chronic inflammatory diseases, in particular CF. One of the main tasks for physicians in CF is adjusting therapy to acute pulmonary complications of chronic inflammation. Identifying acute infectious exacerbations is based on clinical experience, rather depending on subjective impressions than using objective parameters. Consensus is lacking about criteria to define acute episodes. Conventional parameters normally used to identify acute infections, e.g. fever, leukocytosis, CRP, ESR, deterioration of lung function, and sputum cultures, are not always helpful. The chronicity of pulmonary disease together with atypical presenting acute respiratory infections raise major problems for physicians dealing with CF. It would be helpful to have more reliable markers indicating infections to monitor disease and guide therapy. Ideal sensitive markers indicate local bronchial processes before systemic responses occur.

The attempt to find more reliable serum markers for exacerbations was repeatedly made in the past. CRP or ESR have failed to be generally useful in CF exacerbations (Watkin et al.,1994, Pediatr Pulmonol 17: 6-10). More sophisticated potential markers, such as interleukins or tumor necrosis factor (TNF), are not considered as useful tools by all investigators (see e.g. Wolter et al., 1999, Immunol 6: 260-5). Eichler *et al*. proposed human neutrophilic lipocalin as a marker for CF exacerbations (1999, Eur Respir J 14: 1145-9). Sputum levels of various cytokines are detectable, but analysing sputum is very critical (see e.g. Karpati et al., 2000, Scand J Infect Dis 32: 75-9). Reliable examination often requires bronchioalveolar lavage (Smith et al., 1988, J Pediatr 112: 547-54). Exhaled nitric oxide has been shown to be not helpful in CF (Grasemann et al., 1998, Arch Dis Child 78: 49-53).

Also the aetiology and pathogenesis of chronic inflammatory bowel (or intestinal) disease such as Crohn's disease and ulcerative colitis is still poorly understood. Various hypotheses have been proposed to explain the pathophysiology ranging from genetic alterations, dys-regulated immune response against constituents of the normal gut flora or unsuccessful elimination of unknown antigens (Sator, 1997, Am J Gastroenterol 92: 5S-11S). Despite obvious differences in initiating mechanisms, Crohn's disease and ulcerative colitis share common immunological aberrations that constitute a status of ongoing inflammatory processes (Brandzaeg et al., 1997, Springer Semin Immunopathol 18: 555-589). One of the most prominent histological feature that is observed in ulcerative colitis as well as in Crohn's disease is the infiltration of neutrophils into the inflamed mucosa at an early time point of inflammation (Nikolaus et al., 1998, Gut 42: 470-476; Kucharzik et al., 2001, Am J Pathol 159: 2001-2009). Disease activity in inflammatory intestinal disease is linked to an influx of neutrophils into the mucosa and subsequently into the intestinal lumen resulting in the formation of socalled crypt abscesses. Neutrophil migration across intestinal epithelia induces transient opening of intercellular junctions, but does not usually cause morphological discontinuities. (Nusrat et al., 1997, Gastroenterology 113: 1489-1500). One of the possible mediators that has been suggested to induce neutrophil infiltrate during the inflammatory process of inflammatory intestinal disease is epithelial derived interleukin-8 (IL-8) (Imada et al., 2001, Scand J Gastroenterol 36: 854-864; McCormick et al., 1995, J Cell Biol 131: 1599-1608). Activated neutrophils secrete a variety of pro-inflammatory cytokines and chemokines and thereby trigger infiltration of various inflammatory cells including monocytes, macrophages, lymphocytes, and granulocytes (Burgio et al., 1995, Gastroenterology 109: 1029-1038; Reinecker et al., 1993, Clin Exp Immunol 94: 174-181; Mazlam et al., 1994, Gut 35: 77-83; MacDermott et al., 1998, Inflamm Bowel Dis 4: 54-67). One of the most important mediators during the inflammatory process of inflammatory intestinal disease is tumor necrosis factor alpha (TNF alpha) that is expressed in the intestinal mucosa of patients with inflammatory intestinal disease (Murch, 1998, Nutrition 14: 780-783; Breese et al., 1994, Gastroenterology 106: 1455-1466). TNF alpha triggers inflammation via an intracellular nuclear factor kappa B (NF-kappa B) dependent signalling cascade. NF-kappa B plays a key role for downstream processes in chronic inflammation such as inflammatory intestinal disease by controlling transcription of pro-inflammatory cytokine genes (Baldwin, 1996, Annu Rev Immunol 14: 649-683; Rogler et al., 1998, Gastroenterology 115: 357-369). A recent therapeutic schedule to treat active Crohn's disease involves the administration of TNF blocking agents such as anti-TNF antibodies, e.g. infliximab.

Diagnosis of the disease activity of inflammatory intestinal diseases, especially Crohn's disease and ulcerative Colitis, is mainly assessed using clinical observations, e.g. general well-being. Thus, there is a need for sensitive and reliable biological markers for disease activity in order to reliably assess disease activity; however, biological markers tested so far, such as CRP; ESR, leukocyte and platelet counts, were not found to be suitable (Nielsen et al., 2000, Am J Gastroenterol 95:1849-1850).

Human CALGRANULIN C, which is also called S100A12, EN-RAGE, CAAF1 and p6 protein, is a small protein of 92 amino acids which belongs to the family of calcium-binding S100 proteins (Guignard et al., 1995, Biochem J 309: 395-401; US 5,976,832). Homologues of CALGRANULIN C in other species are known from *Bos taurus* (US 5,976,832), pig (Dell'Angelica, 1994, J Biol Chem 269: 28929-28936) and rabbit (partial sequence: Yang et al., 1996, J Biol Chem 271: 19802-19809). Like other S100 proteins, it is suggested to play a role in general inflammation, although the role in inflammation within the S100 family is inconsistent in that some of them are inhibiting the function of inflammatory cells while others are activating. It was proposed that CALGRANULIN C plays a proinflammatory role (Donato, 2001, Int J Biochem Cell Biol 33: 637-668; Donato, 1999, Biochim Biophys Acta, 81450:191-231; Yang et al., 2001, JLeukoc Biol 69: 986-994). S100 proteins accumulate at sites of inflammation, and high levels of S100A8 (also referred to as myeloid-related protein 8, MRP8 or calgranulin A) and S100A9 (MRP14 or calgranulin B) are found in inflammatory diseases like rheumatoid arthritis, inflammatory bowel disease, and CF (Golden et al., 1996, Arch Dis Child 74: 136-9; Frosch et al., 2000, Arthritis Rheum 43: 628-37; Roth et al., 2001, Lancet 357:1041). Overexpression of murine S100A8 was detected in a mouse model of CF (Thomas et al., 2000, J Immunol 164: 3870-3877).

CALGRANULIN C is expressed by granulocytes, whereas it's expression by monocytes remains controversial (Vogl et al., 1999, J Biol Them 274: 25291-25296; Hofmann et al. 1999, Cell 97: 889-901; Yang et al., 2001, J Leukoc Biol 69: 986-994; Robinson et al., 2000, Biochem Biophys Res Commun 275: 865-870). It is secreted by activated granulocytes (Boussac et al., 2000, Electrophoresis 21: 665-672). Extracellular functions include potent chemotactic activity comparable to other strongly chemotactic agents (Hofinann et al. 1999, Cell 97: 889-901; Miranda et al., 2001, FEBS Lett 488: 85-90). CALGRANULIN C is a ligand for the receptor for advanced glycation end products (RAGE) expressed on macrophages, lymphocytes, and endothelium (Hofmann et al. 1999, Cell 97: 889-901). Intracellular signalling via protein kinases induces nuclear factor (NF)-kappa B-dependent secretion of different cytokines (Yeh et al., 2001, Diabetes 50: 1495-1504; Lander et al., 1997, J Biol Chem 272: 17810-17814). NF-kappa B plays a central role in the pathogenesis of synovitis in rheumatoid arthritis and psoriatic arthritis (Danning et al., 2000, Arthritis Rheum 43: 1244-1256) Thus, CALGRANULIN C is the first S100 protein for which a convincing receptor model has been described. The name EN-RAGE (for extracellular newly identified RAGE-binding protein) has been proposed to emphasise its central role for a receptor-mediated signalling pathway, which might offer attractive targets for intervention with blocking agents.

Proteins directly or indirectly involved in some inflammatory processes are very common. However, there is a need for diagnostic markers which are specific, in order to discriminate between diseases with similar symptoms, especially SOJRA and bacterial infections, to monitor disease states for adequate treatment, especially vasculitis, in particular Kawasaki disease, and CF, and to determine the risk of relapse for a certain disease, especially JRA, to again determine proper treatment. In particular, diagnosing the disease state by identifying acute excacerbations in chronic inflammatory diseases, especially CF acute exacerbation and diagnosing the disease state by identifying subpopulations of patients, especially subpopulations of vasculitis, in particular Kawasaki disease patients with coronary artery problems, would enable adequate treatment of these diseases.

WO 01/86002 relates to methods for detecting and characterizing psoriasis. A variety of markens is provided, wherein changes in the levels of expression of these markers is correlated with the presence of psoriasis.

EP 0 731 166 relates to calgranulin C as well as to methods for its production.

Zheng Yang et al (J. Leukoc. Biol, vol 69, no. 6, June 2001, pages 986-994) describes a functional study on calgranulin C, which was found in the synovial fluid from rheumatoid arthitis patients.

Hence, there is a need for a reliable diagnostic tool especially in the early stages of an acute inflammatory exacerbation and/or for determining the risk of relapse and/or to discriminate between diseases with similar symptoms in order to apply an appropriate medication.

It is therefore a major object of the present invention, to provide a new method for diagnosing inflammatory diseases by using a reliable marker of inflammation, particularly for diagnosing specific stages of inflammatory diseases and/or for determining the risk of relapse and/or for discriminating between diseases with similar symptoms in order to apply an appropriate medication.

### SUMMARY OF THE INVENTION

The present invention provides a
method for the diagnosis of inflammatory diseases selected from rheumatoid arthritis, seronegative arthritis, juvenile arthritis, and systemic onset juvenile rheumatoid arthritis (SOJRA), comprising the steps of
a) determining the amount and/or concentration of calgranulin C polypeptide in a serum sample from a patient; and
b) comparing the amount and/or concentration of calgranulin C polypeptide determined in said serum sample with the amount and/or concentration of calgranulin C polypeptide as determined in a serum carbol sample
wherein an increase in the amount of calgranulin C polypeptide in the serum sample as compared to the control sample is indicative for said disease.

Further embodiments of the present invention are described in dependent claims 2 to 7.

The amount and/or concentration of CALGRANULIN C polypeptide present in said serum sample is determined. This determination can be achieved via one of several techniques including but in no way limited to: (i) immunohistochemistry of the serum sample utilising antibodies directed to CALGRANULIN C protein(s); (ii) quantitative measurement of CALGRANULIN C proteins in the serum sample; (ii) measurement of the CALGRANULIN C proteins in

In yet another preferred method according to the invention, a specific antibody is used for determining the amount and/or concentration of CALGRANULIN C polypeptide. Preferably, said specific antibody recognises an epitope derived from the amino acid sequence depicted in SEQ ID NO:2. The generation of antibodies and determination of epitopes is well known to the person skilled in the art and can be found in the standard textbook literature in this technical field. Preferably, said antibody is selected from the group comprising polyclonal antiserum, polyclonal antibody, monoclonal antibody, antibody fragments, single chain antibodies an diabodies. Even more preferably, said antibody is used for performing an immunoassay, such as an enzyme immunoassay (EIA), e.g. ELISA, or a immunohistochemical method.

In one particularly preferred method, the target CALGRANULIN C molecules in the serum sample are exposed to a specific antibody which may or may not be labelled with a reporter molecule. Depending on the amount of target and the strength of the reporter molecule signal, a bound target may be detectable by direct labelling with an antibody. Alternatively, a second labelled antibody, specific to the first antibody, is exposed to the target-first antibody complex to form a target-first antibody-second antibody tertiary complex. The complex is detected by the signal emitted by the reporter molecule.

By "reporter molecule" as used in the present specification, is meant a molecule which, by its chemical nature, provides an analytically identifiable signal which allows the detection of antigen-bound antibody. Detection may be either qualitative or quantitative. The most commonly used reporter molecules in this type of assay are either enzymes, fluorophores or radionuclide containing molecules (i.e. radioisotopes) and chemiluminescent molecules.

In the case of an EIA, an enzyme is conjugated to the second antibody, generally by means of glutaraldehyde or periodate. As will be readily recognised, however, a wide variety of different conjugation techniques exists, which are readily available to the skilled artisan. Commonly used enzymes include horseradish peroxidase, glucose oxidase, beta-galactosidase and alkaline phosphatase, amongst others. The substrates to be used with the specific enzymes are generally chosen for the production, upon hydrolysis by the corresponding enzyme, of a detectable color change. Examples of suitable enzymes include alkaline phosphatase and peroxidase. It is also possible to employ fluorogenic substrates, which yield a fluorescent product rather than the chromogenic substrates noted above. In all cases, the enzyme-labelled antibody is added to the first antibody hapten complex, allowed to bind, and then the excess reagent is washed away. A solution containing the appropriate substrate is then added to the complex of antibody-antigen-antibody. The substrate will react with the enzyme linked to the second antibody, giving a qualitative visual signal, which may be further quantified, usually spectrophotometrically, to give an indication of the amount of hapten which was present in the sample.

Alternatively, fluorescent compounds, such as fluorescein and rhodamine, may be chemically coupled to antibodies without altering their binding capacity. When activated by illumination with light of a particular wavelength, the fluorochrome-labeled antibody absorbs the light energy, inducing a state to excitability in the molecule, followed by emission of the light at a characteristic wavelength visually detectable with a light microscope. As in the EIA, the fluorescent labelled antibody is allowed to bind to the first antibody-hapten complex. After washing off the unbound reagent, the remaining tertiary complex is then exposed to the light of the appropriate wavelength and the fluorescence observed indicates the presence of the hapten of interest. Immunofluorescene and EIA techniques are both very well established in the art and are particularly preferred for the present method. However, other reporter molecules, such as radioisotope, chemiluminescent or bioluminescent molecules, may also be employed.

Finally, it is possible to perform an analysis of the expression of CALGRANULIN C by proteolytic cleavage of the protein, e.g. using a protease and subsequent analysis by mass spectroscopy, e.g. MALDI-TOF. Such methods are also known to the person skilled in the art.

As a next step, the amount and/or concentration of CALGRANULIN C polypeptide determined in said serum sample is compared with the amount and/or concentration of CALGRANULIN C polypeptide as determined in a control sample Such comparison will be based on the information obtained in the above determination of the amount and/or concentration of CALGRANULIN C. The data or information can be present in both written or electronic form, i.e. on a suitable storage medium. The comparison can either be performed manually and individually, i.e. visually by the attending physician or the scientist in the diagnostic facility, or done by a suited machine, like a computer equipped with a suitable software. Such equipment is preferred for routine screening, e.g. in an intensive care unit of a hospital. High-throughput environments (i.e. assemblies) for such methods are known to the person skilled in the art and also described in the standard literature.

As an optional step, the amounts and/or concentrations of at least one conventional inflammatory marker polypeptide and/or nucleic acids encoding the polypeptide present in said serum sample and in said control sample can be determined.

By "conventional marker" or "conventional inflammatory marker" as used in the present specification, is meant a marker other than CALGRANULIN C that is induced in the course of an inflammatory disease. According to a preferred method according to the present invention, said conventional inflammatory marker is selected from the group consisting of CRP, human neutrophilic lipocalin, ESR, soluble receptors, e. g. Fas, and cytokines. Such conventional markers provide a simple "plus/minus" or "inflammation-yes/no" information with respect to an inflammation. For:the purpose of the present invention, these markers provide both an internal control and fixed point in time, at which the inflammation is, for example, present and acute. The comparison of CALGRANULIN C with the conventional marker and/or the expression in the control sample will thus provide additional viable information for the diagnosis, monitoring, treatment, and especially for the prevention of an inflammatory disease. During the experiments performed in the course of completion of the present invention, the inventors found that CALGRANULIN C can be used as an early inflammatory marker, whose induction (or onset) occurs much earlier and to an extraordinary high extent in contrast to other conventional markers. This allows for a much earlier and thus more efficient diagnosis of stages of inflammatory diseases and, in turn, for a much earlier, efficient and less time consuming treatment of inflammatory diseases. The use of the inventive marker, and in particular in connection with a conventional inflammatory marker increases the comfort for the patients that experience the inflammation.

In addition, the high induction provides for a clear diagnosis and thus a very precise monitoring of the stages of inflammatory diseases. Inflammatory diseases which can be diagnostically followed, are rheumatoid arthritis, and systemic onset juvenile rheumatoid arthritis (SOJRA, Still's disease).

By "stages of inflammatory diseases" or "stages of diseases" as used in the present specification, is meant the different phases, of the course of an inflammatory disease. Such phases include the early, acute, and regressive phase during the time period during which a patient experiences said disease. Stages of a disease include also an exacerbation of a present disease, secondary infections to an already existing disease, an acute inflammation above the background of a chronic inflammation, an acquired infection on the background of a chronic inflammatory disease, the risk of relapse, and/or discriminating between diseases with similar symptoms.

Thus in one aspect of the method according to present invention, the inflammatory disease is an acquired infection on the background of a chronic inflammatory disease. In yet another aspect of the method according to present invention, the inflammatory disease is an exacerbation of an already present disease.

Preferably, the method according to present invention is used for for determining the risk of relapse and/or for discriminating between diseases with similar symptoms.

Stages of diseases in general, and in particular inflammatory diseases; are frequently diagnosed based on clinical symptoms that are observed by the attending physician. Based on the diagnosis, the stage (in most of the cases corresponding to the severity of the disease) is evaluated. Nevertheless, in addition to the "classical" diagnosis, which is usually based on visual inspection and conventional blood inflammation markers, in recent diagnosis, the analysis of inflammatory markers has become an additional tool for the analysis of the stages of inflammatory diseases. A prominent conventional marker of this family of diagnostically suitable markers is C-reactive protein (CRP). Nevertheless, this marker is quite slow in its response to an inflammation and not induced in all cases in a very high ratio, compared to its non-inflammation expression. For example, the stages of a disease can be designated as acute outbreak, exacerbation, relief, and include fever and other symptoms. Furthermore, the present invention allows the diagnosis of a disease even in patients showing a healthy appearance, but having a risk of relapse for a disease. By the term "relapse" is meant that in contrast to a "naive" patient for the infection, the person already experienced at least one stage of the respective inflammatory disease. This includes also the distinction between diseases that were experienced and are newly acquired.

One example for the analysis and grading of stages of a disease is described here (in a not limited manner) in the case of rheumatoid arthritis. Rheumatoid arthritis can last for many years. The progression (i.e. stages or phases) of the disease is categorised by five different stages of development. Stage I: You will not experience any of the common signs or symptoms, although you may have a flu-like illness. Stage II: You experience mild pain and swelling in small joints such as your hands, wrists, knees and feet. You may also experience a general, continuing physical discomfort. X-rays of your joints will appear to be normal at this stage. Stage III: Your affected joints are warm and swollen. You also experience stiffness in the morning, a limitation of motion in affected joints, and general and ongoing physical discomfort and weakness. Stage IV: The symptoms you experienced in Stage III will become more pronounced. Stage V: Symptoms are more pronounced than in Stage IV. You will most likely experience the loss of function of the joints affected. Often deformity occurs. During this stage of the disease, the bone around the joint erodes and ligaments are stretched. Also, additional complications may occur such as tendon rupture, leg ulcers, Sjögren's syndrome and carpal tunnel syndrome.

In yet another aspect of the present invention, the method according to the present invention comprising determining the amount and/or concentration of CALGRANULIN C polypeptide involves determining the amount and/or concentration of CALGRANULIN C polypeptide as a local marker. By "local marker" as used in the present specification, is meant a marker that is produced directly at the site of the inflammatory disease. A local marker thus stands in contrast to conventional markers that are produced as a general response to an infection and/or inflammatory stimulus. Such markers include, amongst others, CRP, human neutrophilie lipocalin, ESR, soluble receptors, like Fas, and cytokines. In contrast, CALGRANULIN C can be shown in synovial fluid, indicating its localised production. Local markers have particular advantages in the analysis of a potential relapse of a disease, as could be shown in the present case with JRA-patients that seemed to be healthy, yet having a increased risk of relapse for said disease. Nevertheless, the use of CALGRANULIN C as marker shall not be limited to localised inflammations, as this marker (although at a slightly later point in time) is present also in the, for example, serum of the patients.

As mentioned above, the method of the present invention can form the basis for a method of treatment of an inflammatory disease in a subject (i.e. a mammal) in need thereof. Thus, described is a method of treatment of an inflammatory disease in a mammal in need thereof, comprising the steps of: a) Performing steps a) to c) according to the method of the present invention as indicated above; and b) medical treatment of the mammal in need of said treatment; wherein said medical treatment is based on the stage of the disease to be treated. By "medical treatment" or "medication" as used in the present specification, is meant the use of medicaments, therapeutics and/or exercises in order to support and accelerate the regression of the symptoms of the inflammation. Medical treatment is classically performed using drugs or combinations of drugs that are specifically prescribed by the skilled attending physician. Nevertheless, the term medication shall not be limited to the ingestion of drugs, but includes all possible ways of treatment that will show a benefit for the subject to be treated.

Due to the fact that the medication is based on the stage of the disease to be treated, the attending physician will usually alter the treatment scheme and/or the collection of drugs prescribed and used in order to treat the inflammatory disease. This alteration, which is based on the results of the diagnosis according to the method of the present invention, will allow for the treatment to be earlier, more specific, and thus more effective for the patient. Furthermore, an early medication will save costs, reduce the need to stay in clinics and allow for an ambulant treatment at home, which will increase the comfort of the patient even further. The alterations of the treatment scheme are based on the diagnosis according to the present invention, which, in this case, can be described by "monitoring" of the stages of the disease and the success of a medication. Furthermore, severe side effects that occur during treatment with chemotherapeutics, e.g., MTX, can be avoided in cases, in which the risk for the patients for a relapse was diagnosed as low or not present at all.

In a preferred method of treatment as described the conventional inflammatory marker is selected from the group consisting of CRP, human neutrophilic lipocalin, ESR, soluble receptors, e. g. Fas, and cytokines. In most cases, such conventional markers provide a simple "plus/minus" or "inflammation-yes/no" information with respect to an inflammation. For the purpose of the present invention, these markers provide both an internal control and fixed point in time, at which the inflammation is, for example, present and acute. The comparison of CALGRANULIN C with the conventional marker and/or the expression in the control sample will thus provide additional viable information for the diagnosis, treatment, and especially for the prevention of an inflammatory disease.

Also described is a method of prevention of an inflammatory disease in a mammal in need thereof, comprising the steps of: a) Performing steps a) to c) according to claim 1; and b) medical treatment of the mammal in need of said treatment; wherein said medical treatment is based on the stage of the disease to be prevented. In the context of the present invention, the term "prevention" is meant as a specific treatment of a disease that does not yet exhibit "classical" symptoms (like those mentioned above, e.g. induction of conventional markers), but can be diagnosed by the method according to the present invention above, e. g. relapse risk. Based on the information of the diagnosis according to the present invention, the attending physician will usually begin (e.g. "alter") with a treatment scheme and/or the collection of drugs prescribed and used in order to prevent (treat) the inflammatory disease. This "early onset"-treatment, which is based on the results of the diagnosis according to the method of the present invention, will allow for a more effective prevention than with conventional markers, thus allowing a more effective prevention for the patient. Furthermore, an early medication will save costs, reduce the need to stay in clinics and allow for an ambulant treatment at home, which will increase the comfort of the patient even further. Finally, the possibility to diagnose a risk for a relapse of a disease using the method of the invention allows for a treatment only in cases in which such treatment is necessary, thus avoiding and/or reducing side effects for patients that are treated, for example, treated with chemotherapeutics like, e.g. MTX, and/or with an antibody like, e.g. infliximab.

In a preferred method of prevention the conventional inflammatory marker is selected from the group consisting of CRP, human neutrophilic lipocalin, ESR, soluble receptors, e.g. Fas, and cytokines. Such conventional markers provide a simple "plus/minus" or "inflammation-yes/no" information with respect to an inflammation. For the purpose of the present invention, these markers provide both an internal control and fixed point in time, at which the inflammation is, for example, present and acute. The comparison of CALGRANULIN C with the conventional marker and/or the expression in the control sample will thus provide additional viable information for the diagnosis, treatment, and especially for the prevention of an inflammatory disease.

In a preferred method of prevention the inflammatory disease is a localised inflammatory disease. Such localised inflammations stand in contrast to systemic infections and/or inflammations, like, for example, sepsis or bacterial toxic shock syndrome. In these cases, the prevention of inflammation will have the additional benefit, to prevent a spreading of the local infection and thus the development from a local towards a systemic (i.e. not localised) inflammation. Nevertheless, the use of CALGRANULIN C as marker shall not be limited to- localised inflammations, as this marker (although at a slightly later time) is present also in the, for example, serum of the patients.

### DETAILED DESCRIPTION OF PREFERRED EMBODIMENTS

The invention shall now be further described by the following examples with respect to the attached figures. All examples are provided by way of example only, without any intended limitation of the scope of the invention.

Examples described in the following paragraphs which do not relate to the invention as claimed in claims 1 to 7 merely serve as further illustration.

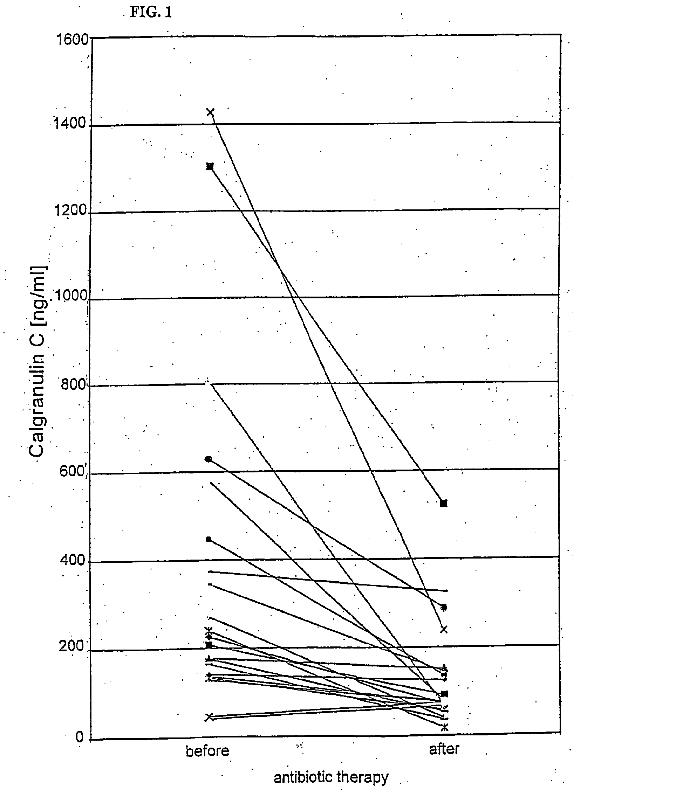

Figure 1: shows CALGRANULIN C concentrations in CF patient sera before and after antibiotic treatment. *Figure 1* thus shows, that the CALGRANULIN C concentration in serum of CF patients is decreased upon treatment with antibiotics.

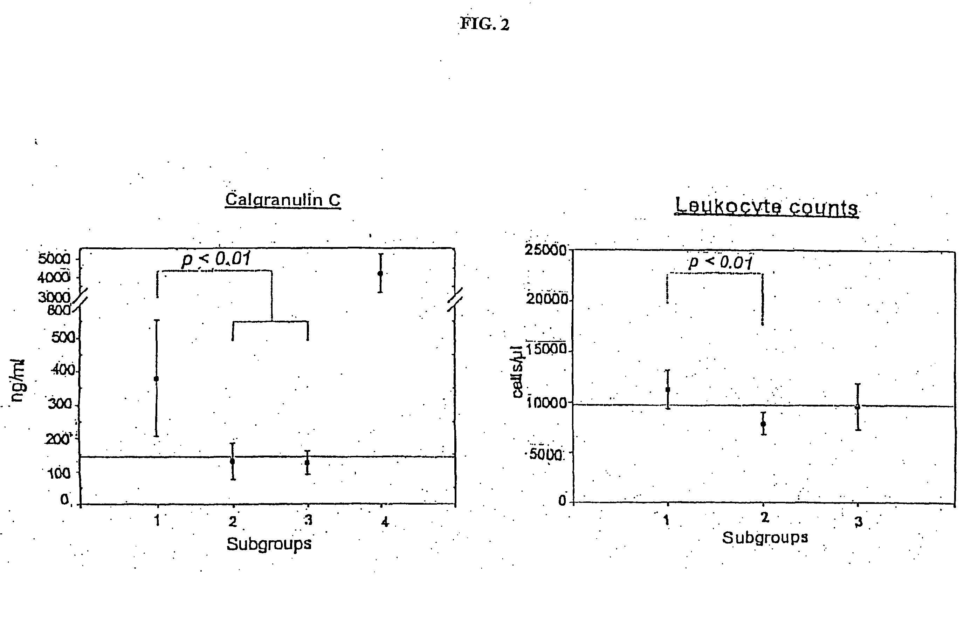

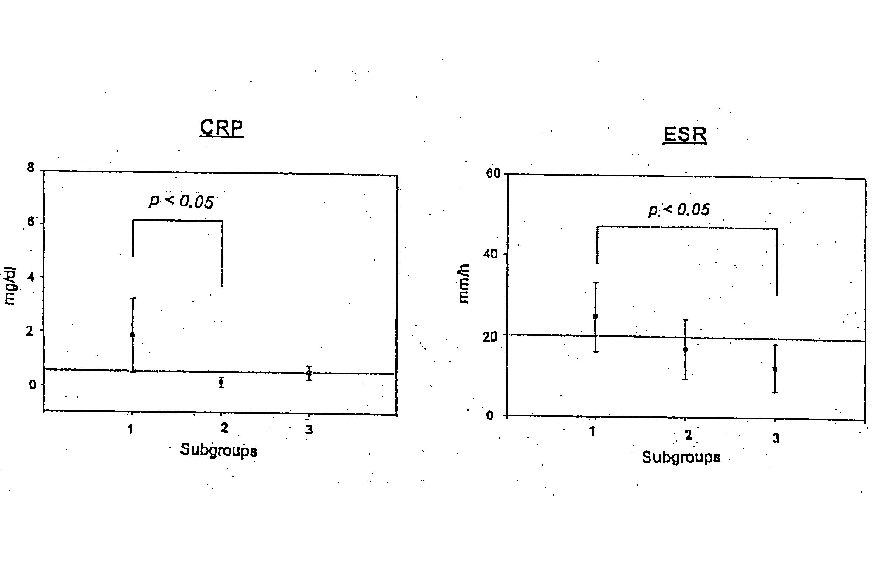

Figure 2: shows a comparison of markers for inflammation (CALGRANULIN C, Leukocyte counts, CRP, and ESR) in CF patients. Subgroups: 1) CF patients with acute exacerbation before start of antibiotic treatment (n=21) 2) CF patients at the end of antibiotic therapy (n=21) 3) CF out-patients (n=20); 4) sputum of CF patients with acute exacerbation (n=10). CALGRANULIN C concentration was measured in serum (1-3) and sputum (4). Data are expressed as means, error bars indicate 95% confidence interval. Grey lines indicate upper limit of normal range. *Figure 2* thus demonstrates CALGRANULIN C as the most sensitive marker of acute CF exacerbation compared to leukocyte counts, CRP and ESR. Only CALGRANULIN C concentrations show significant differences between acute exacerbation before start of antibiotic treatment and both the situations after antibiotic treatment and in out-patients.

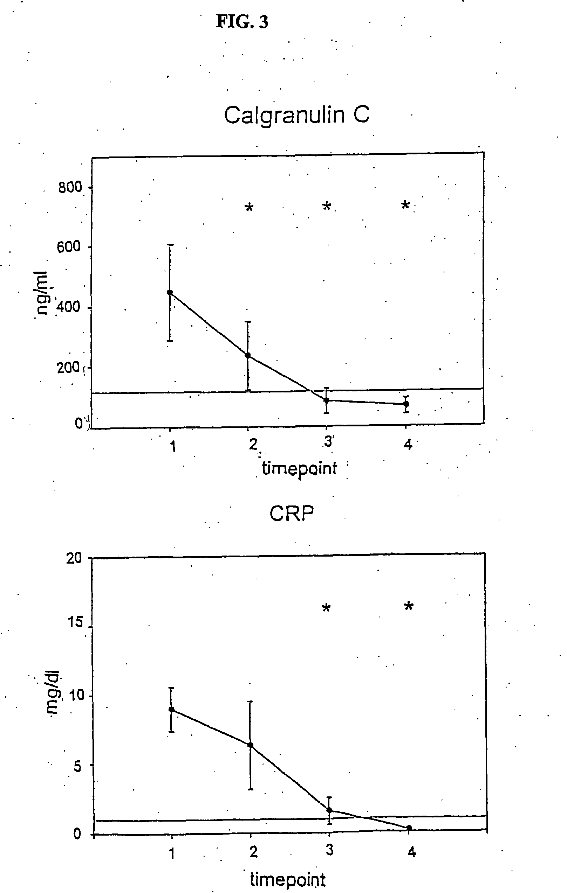

Figure 3: shows serum markers CRP and CALGRANULIN C in the monitoring of Kawasaki disease. Indicated time points 1) initially before start of therapy 2) after intravenous gammaglobulin 3) after 2 weeks 4) in remission. Data are expressed as means, error bars indicate 95% confidence interval. Grey lines indicate upper limit of normal range. Asterisks indicate statistical significance. *Figure 3* thus demonstrates, that CALGRANULIN, compared to CRP, is suitable to indicate the difference between the inflammatory state of disease before and after gammaglobulin treatment.

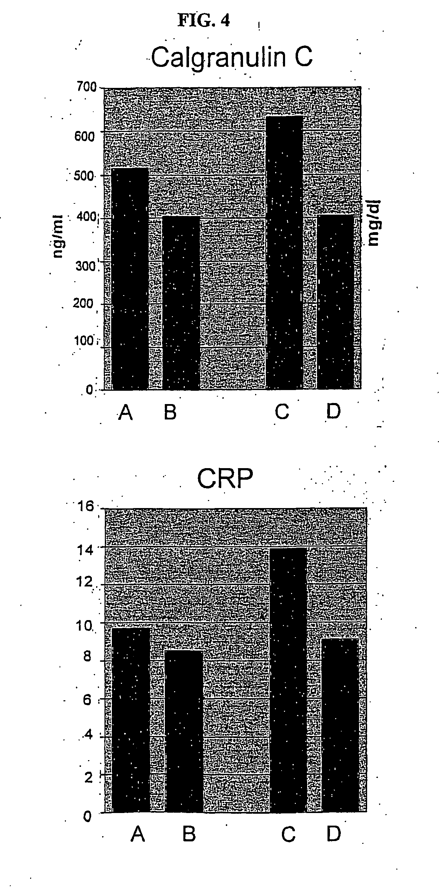

Figure 4: shows mean serum levels for different groups of patients with Kawasaki disease. A) initial level in patients with coronary artery lesions B) initial level in patients without coronary artery lesions C) maximal level in patients with coronary artery lesions D) maximal level in patients without coronary artery lesions. *Figure 4* thus demonstrates CALGRANULIN C as being superior to CRP in identifying cases at high risk for coronary artery lesions.

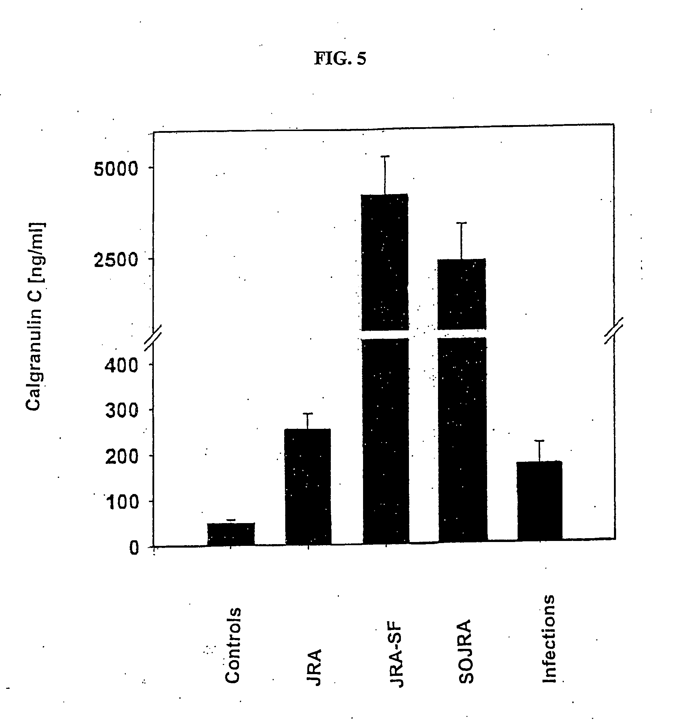

Figure 5: shows serum concentrations of CALGRANULIN C in control persons (Controls), JRA patients (JRA), SOJRA patients (SOJRA), and patients suffering from bacterial infections, as well as CALGRANULIN C concentration in the synovial fluid of JRA patients (JRA-SF). *Figure 5* thus demonstrates serum CALGRANULIN C as a highly sensitive marker which enables discrimination between SORJA and JRA or bacterial infections.

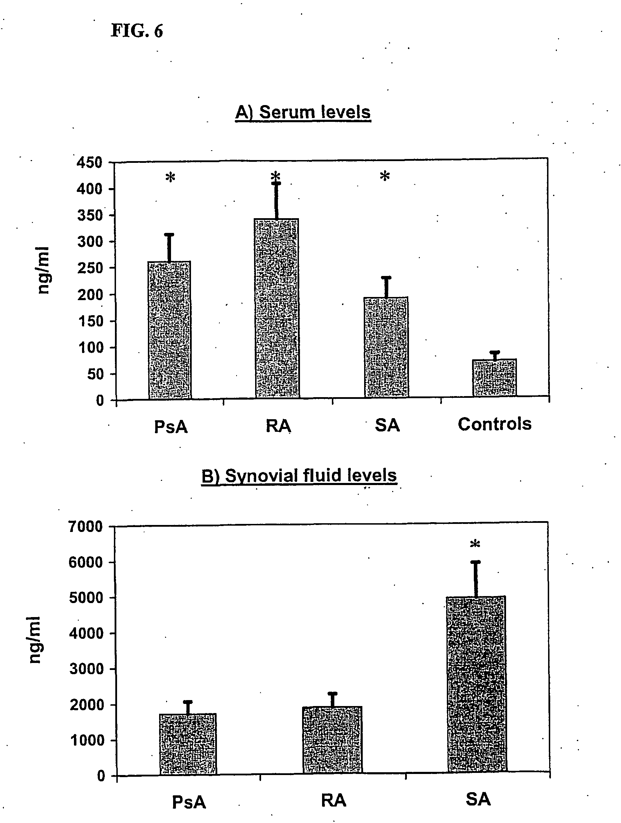

Figure 6: shows a comparison of CALGRANULIN C concentrations in serum (A) and synovial fluid (B) in patients with psoriatic arthritis (PsA), rheumatoid arthritis (RA) and seronegative arthritis (SA) and in controls, respectively. (* p < 0.05). *Figure 6* thus demonstrates CALGRANULIN C as a serum marker indicating arthritic inflammation.

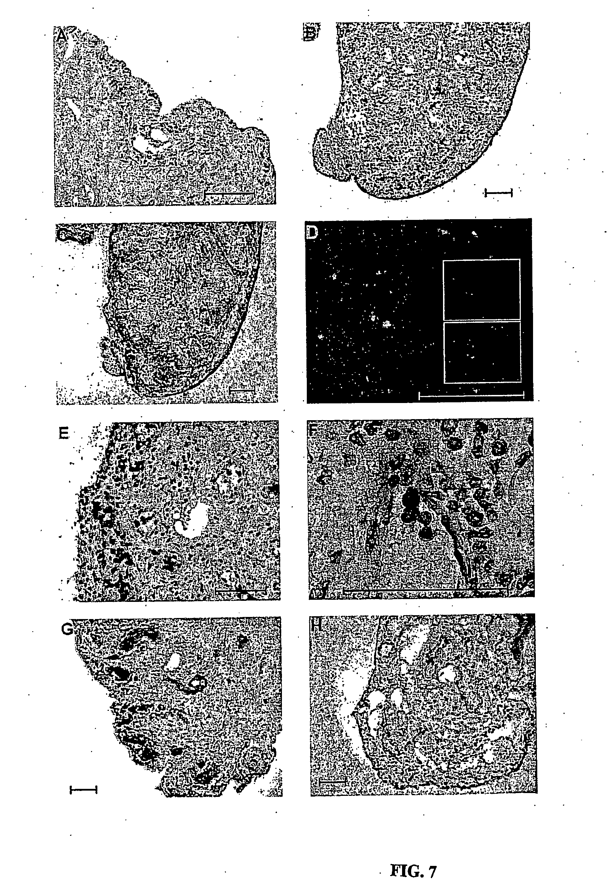

Figure 7: shows the expression of CALGRANULIN C in synovial biopsies. Virtually no CALGRANULIN C was found in synovial tissue of controls without arthritis (A), whereas CALGRANULIN C was extensively expressed in inflamed synovial tissue of patients with rheumatoid arthritis (B). Expression pattern in seronegative arthritis was similar to rheumatoid arthritis (not shown). CD163-positive macrophages were the most abundant cell type in infiltrates but showed a different distribution than CALGRANULIN C-positive cells (C). Immunofluorescence microscopy of double-labelling studies clearly proved expression of CALGRANULIN C by infiltrating CD15-positive neutrophils. Double-labelled cells appear yellow due to the summation of colours (D). The inserted small images in *Figure* 7D show emission at a single wavelength for either of both fluorochromes with anti-CALGRANULIN C-Texas Red (red; upper image) and a-CD15-FITC (green; lower image). In psoriatic arthritis, CALGRANULIN C was expressed predominantly in the sub-lining layer with a perivascular pattern. The expression of CALGRANULIN C was most impressive around small blood vessels and in perivascular neutrophilic infiltrates (E). CALGRANULIN C was expressed by granulocytes adherent to vascular endothelium and infiltrating the interstitial tissue. CALGRANULIN C seemed to be released upon contact of neutrophils with the endothelium (F). Strong CALGRANULIN C expression was found in synovial tissue of patients with psoriatic arthritis before MTX treatment (G), while it was nearly undetectable in synovia of the same patients after effective MTX treatment (H). Scale bars, 100 µm. *Figure* 7 thus demonstrates CALGRANULIN C as a good marker for local arthritic inflammation using synovial biopsies.

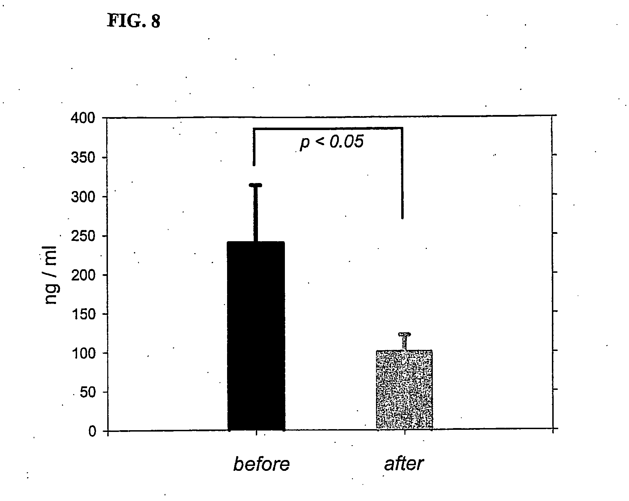

Figure 8: shows serum concentrations of CALGRANULIN C in patients with psoriatic arthritis in active disease at initial presentation and after MTX treatment. *Figure 8* thus demonstrates serum CALGRANULIN C as a highly sensitive marker, which enables monitoring (by measuring) the success of the treatment in psoriatic arthritis.

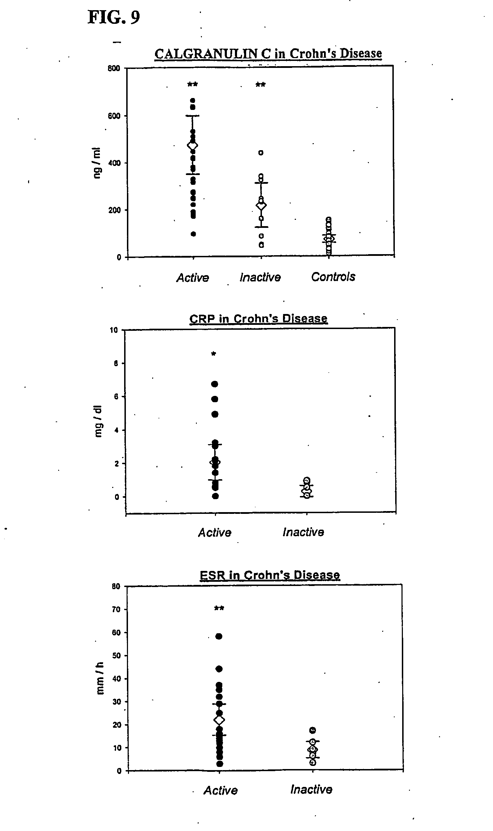

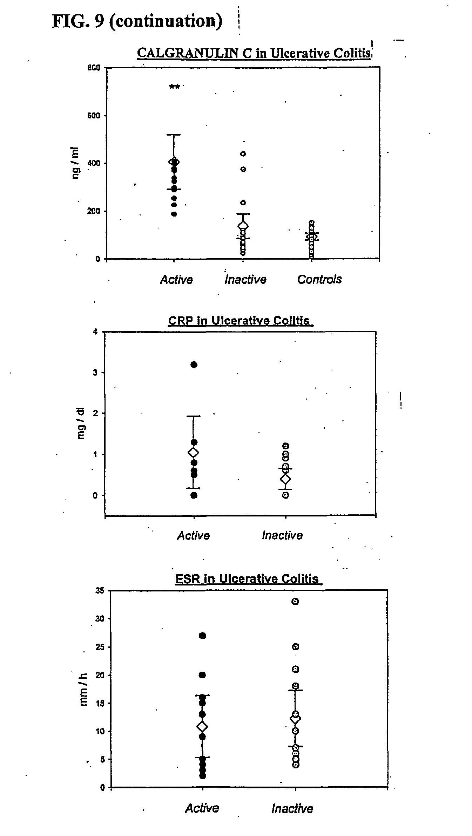

Figure 9: shows serum concentrations of CALGRANULIN C, CRP, and ESR in Crohn's disease and ulcerative colitis. CALGRANULIN C was measured in 40 Crohn's disease patients, 34 ulcerative colitis patients, and 30 healthy controls. CRP was measured in 15 Crohn's disease patients and 26 ulcerative colitis patients. ESR was measured in 28 Crohn's disease patients and 26 ulcerative colitis patients. Circles show individual serum levels of patients. Diamonds indicate mean values. Error bars indicate 95% confidence intervals (* p<0.05, * * p<0.01). *Figure 9* thus demonstrates CALGRANULIN C as a good serum marker for active inflammation in Crohn's disease and ulcerative colitis, and moreover, CALGRANULIN C is a superior marker for disease activity in ulcerative colitis.

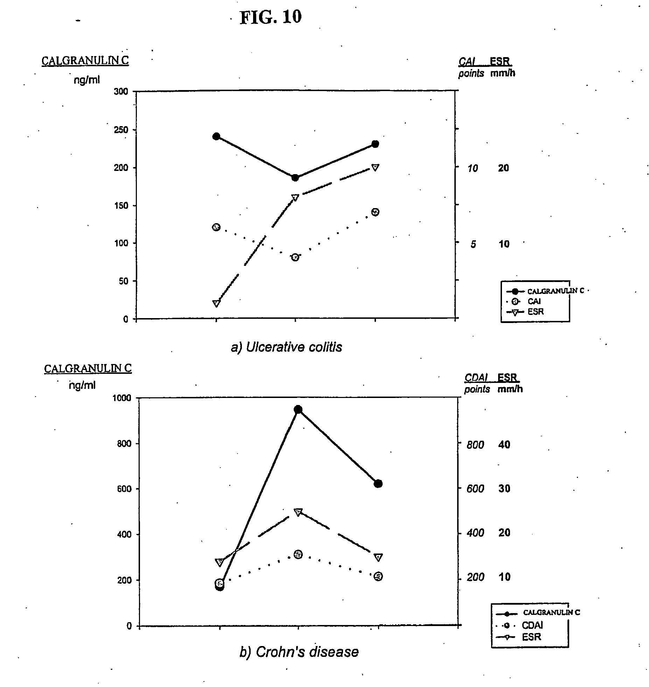

Figure 10: Individual follow-up serum CALGRANULIN C levels and ESR data. Individual courses of CALGRANULIN C, ESR and CAI/CDAI in a patient with ulcerative colitis (a) and Crohn's disease (b), respectively. Data are representative of 10 patients with inflammatory intestinal disease. *Figure 10* thus demonstrates a good correlation of CALGRAULIN C serum concentrations and disease activity.

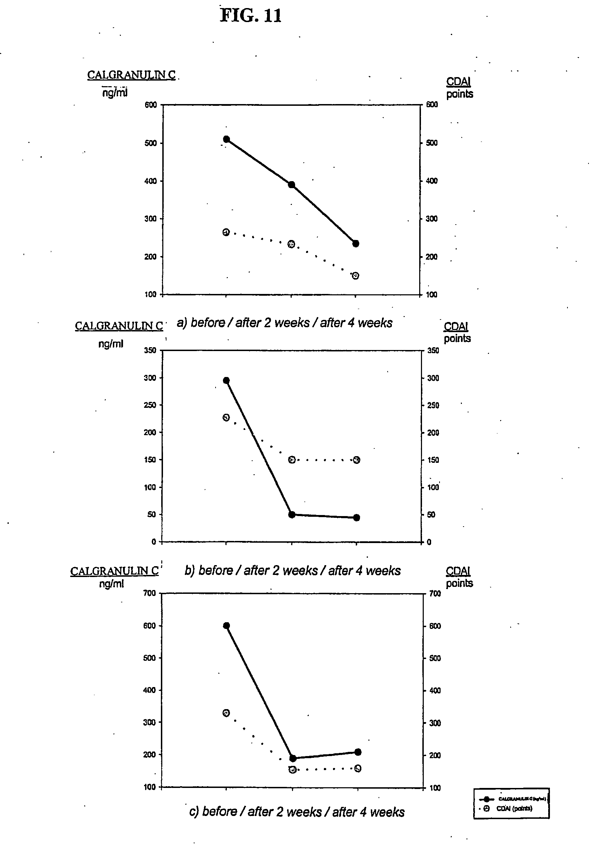

Figure 11: CALGRANULIN C serum levels in patients with Crohn's disease due to infliximab treatment. Individual courses of CALGRANULIN C and CDAI in 3 patients before, 2 weeks, and 4 weeks after treatment with infliximab (a-c). *Figure 11* thus demonstrates a good correlation of CALGRAULIN C serum concentrations and disease activity.

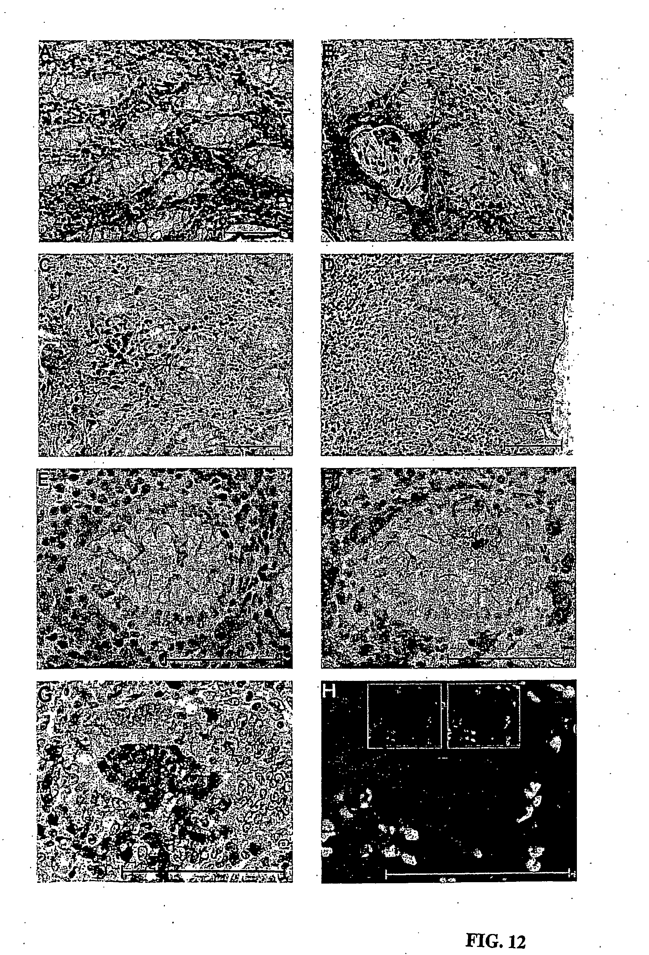

Figure 12: Expression of CALGRANULIN C in tissue from patients with active Crohn's disease or ulcerative colitis. Immunohistochemical staining showed an extensive expression of CALGRANULIN C in inflamed colonic tissue of patients with active Crohn's disease (A). CALGRANULIN C-positive cells surrounded granulomatous lesions in Crohn's disease (B). Similar local expression of CALGRANULIN C was found in ulcerative colitis (C). Numerous CALGRANULIN C-positive cells assembled in crypt abscesses in ulcerative colitis (D). Staining of serial sections revealed a co-localisation of CALGRANULIN C-positive cells (E) and CD15-positive cells (F). In destructive crypt abscesses CALGRANULIN C-positive neutrophils transmigrated through the epithelium into the lumen (G). Immunofluorescenee microscopy of double-labelling studies with anti-CALGRANULIN C-Texas Red (red) and a-CD15-FITC (green) clearly proved expression of CALGRANULIN C by infiltrating CD 15-positive neutrophils (H). Double-labelled cells appear yellow due to the summation of colors. The small inserted figures in (H) show emission at a single wavelength for either of both fluorochromes. Scale bars indicate 100 µm. *Figure 12* thus demonstrates CALGRANULIN C as a good marker for local inflammation of intestinal tissue.

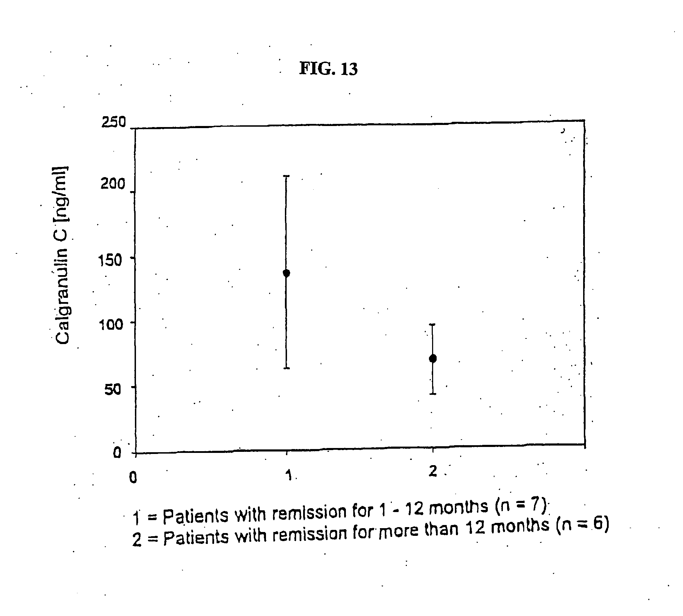

Figure 13: shows CALGRANULIN C serum concentrations of JRA patients in remission without any clinical or laboratory signs of residual inflammatory activity. Group 1 patients (1 on X-axis), which relapsed within 12 months after discontinuation of MTX treatment had significantly higher CALGRANULIN C concentrations in the serum than Group 2 patients (2 one X-axis), which showed remission for more than 12 months. *Figure 13* thus shows CALGRANULIN C is suitable as marker for the relapse risk of JRA patients in remission.

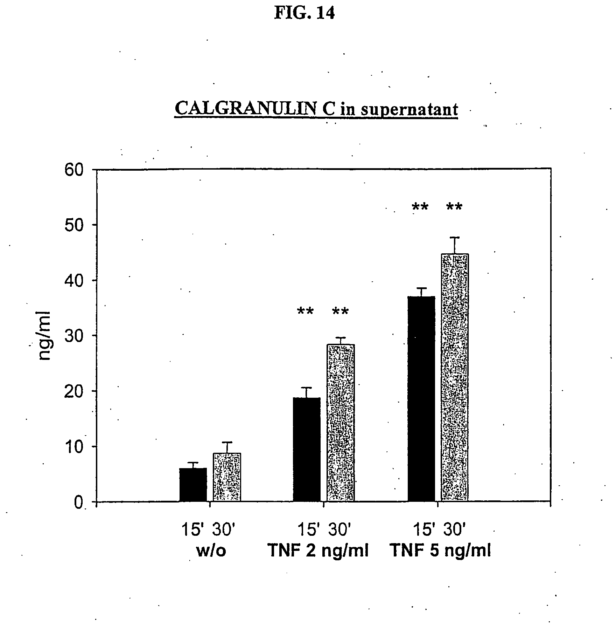

Figure 14: shows CALGRANULIN C concentrations in supernatant of neutrophils after stimulation with TNF alpha. Cells were either left untreated (w/o) or stimulated with TNF alpha for 15 and 30 minutes, respectively. (** p<0.01; n=3). *Figure 14* thus shows CALGRANULIN C secretion from stimulated human neutrophils.

SEQ ID NO:2 depicts the CALGRANULIN C polypeptide sequence, and SEQ ID NO: depicts the CALGRANULIN C nucleic acids sequence encoding the polypeptide.

Surprisingly, it could be shown that polyclonal affinity-purified rabbit-antisera directed against human CALGRANULIN C are useful in a method for diagnosing inflammatory diseases, particularly for diagnosing specific stages of inflammatory diseases and/or for determining the risk of relapse and/or for discriminating between diseases with similar symptoms in order to apply an appropriate medication.

CALGRANULIN C polypeptide according to SEQ ID NO:2 and/or nucleic acids encoding this according to SEQ ID NO:1 and/or an antibody directed against this polypeptide were surprisingly found to be useful for these specific diagnosing needs.

The results presented in the attached figures and discussed in the examples below indicate that CALGRANULIN C is a potent marker for e.g. acute CF exacerbation. CALGRANULIN C serum concentrations are significantly raised in CF patients with exacerbation compared to healthy controls. Furthermore, serum levels correlated with disease activity in individual patients. In all patients, CALGRANULIN C concentrations decreased during antibiotic therapy (*Figure 1*). Even in the four cases with initial serum level inside the normal range, a decrease was detected, possibly indicating that personal profiles might be more useful than single serum tests. CALGRANULIN C is a more sensitive indicator for acute exacerbation than the conventional markers CRP, ESR, and leukocyte counts (*Figure 2*). It is the only parameter with highly significant differences between patients with acute exacerbation before treatment and after treatment, as well as between patients with acute exacerbation and CF out-patients, respectively.

Furthermore, CALGRANULIN C is a potent marker for monitoring the course of vasculitis, in particular Kawasaki disease (*Figure 3*), and for the prognosis of patients with additional artery lesions (*Figure 4*).

CALGRANULIN C is also a systemic marker for the disease activity in inflammatory arthritis diseases (*Figures 5**,* *6**,* *8**,* and *13*) as well as in inflammatory intestinal diseases (*Figures 9*, *10*, and *11*).

In addition, CALGRANULIN C is a marker for detection of local inflammation when using biopsies and tissue specimens, respectively (*Figures 7* and *12*).

Eventually, CALGRANULIN C is a potent marker for discriminating an acute inflammation due to infection from the basic chronic inflammatory disease.

### Example 1: Identification of human CALGRANULIN C as advantageous marker for acute exacerbations in cystic fibrosis (CF) patients

### Preparation of CALGRANULIN C

CALGRANULIN C was isolated from human granulocytes as described in detail previously (Vogl et al., 1999, J Biol Chem 274: 25291-25296; van den Bos, 1998, Prot Expr Purif 13: 313-318).

### Preparation of anti-CALGRANULIN C antisera

Polygonal affinity-purified rabbit-antisera directed against human CALGRANULIN C (anti-CALGRANULIN C) were prepared as reported before (Vogl et al., 1999, J Biol Chem 274: 25291-25296, van den Bos et al., 1998, Protein Expr Purif 13:313-8). Monospecificity of rabbit anti-human CALGRANULIN C antibody was analysed by immunoreactivity against purified human and recombinant CALGRANULIN C, and westemblot analysis of lysates of granulocytes.

### Determination of CALGRANULIN C concentrations by sandwich ELISA

Concentrations of CALGRANULIN C in the serum of patients were determined by a double sandwich enzyme linked immunosorbent assay (ELISA) system. Flat-bottom 96-well microtiter plates (Maxisorp; Nunc, Roskilde, Denmark) were coated at 50 µl/well with 10 ng/well of anti-CALGRANULIN C in 0.1 M sodium carbonate buffer, pH 9.6; incubated for 16 h at 4°C; washed three times with phosphate buffer saline and 0.1 % Tween 20, pH 7.4 (wash buffer); and blocked with wash buffer containing 0.25% bovine serum albumin (block buffer) for 1 h at 37°C. Plates were washed once with wash buffer and 50 µl of samples with varying dilutions in block buffer were added for 1 h at room temperature. The ELISA was calibrated with purified CALGRANULIN C in concentrations ranging from 0.016 to 125 ng/ml. The assay has a linear range between 0.5 and 10 ng/ml and a sensitivity of <0,5 ng/ml. After 3 washes, 20 ng/well of biotinylated rabbit anti-human CALGRANULIN C was added and incubated for 30 min at 37°C. Plates were washed three times and incubated with streptavidine-horseradish peroxidase conjugate (1:5000 dilution; Pierce, Rockford, Illinois, USA) for 30 min at 37°C. After washing three times, plates were incubated with ABTS (2,2'-azinobis(3-ethylbenzthiazoline sulfonic acid); Roche Diagnostics, Mannheim, Germany) and H₂O₂ (10 mg ABTS and 10 µl H₂O₂ (30%) in 25 ml 0.05 M citrate buffer, pH 4.0) for 20 min at room temperature. Absorbency at 405 nm was measured with ELISA-reader (MRX microplate reader, Dynatech Laboratories, St Peter Port, Guernsey, UK).

### Patients and healthy controls

CALGRANULIN C serum concentrations of 17 CF in-patients (9 boys, 8 girls; the mean age at the time of entry into the study was 21,1 years, range 10-35 years), who received intravenous antibiotic therapy upon 21 courses of acute exacerbation at the beginning and at the end of the antibiotic treatment, were determined. The mean duration of hospitalisation for the actual therapy was 2 weeks. Main reasons for hospitalisation were global deterioration of well-being, excessive production of viscous sputum, and increase of productive coughing.

18 CF out-patients (10 boys, 8 girls; mean age 21,8 years with range 8-31 years) without acute exacerbation, who underwent taking blood sample at 20 occasions for other reasons, were investigated for the same inflammatory parameter and for the detection of CALGRANULIN C. We analysed sputum samples of 5 CF-patients with acute exacerbation.

The serum levels of CALGRANULIN C in 18 healthy adults (mean age 31.9; range 19-43) and 16 children without signs of inflammation (mean age 10.9; range 3-17) were estimated. Altogether, 34 normal controls (mean age 22.0; range 3-43) were investigated.

### Statistical analysis

Students T test was performed to determine differences of CALGRANULIN C expression between distinct categories. Data are expressed as mean ± SD. *P* values greater 0.05 were considered to be not significant.

### Results of CALGRANULIN C analysis

Normal CALGRANULIN C means were 64 ± 36 ng/ml for healthy adult controls and 50 ± 32 ng/ml for healthy children. Overall mean in healthy controls was 57 ng/ml. There were no significant differences for age or gender distribution.

CF patients with acute exacerbation had significantly elevated CALGRANULIN C serum levels (mean 381 ng/ml, range 40-1429 ng/ml; p<0.01). In 17 of 21 cases (81%) CALGRANULIN C serum levels were above normal mean plus two standard deviations. After 2 weeks of intravenous antibiotic therapy, mean CALGRANULIN C level in these patients decreased to 130 ng/ml (range 17-524 ng/ml). The mean CALGRANULIN C level for CF out-patients without exacerbation was 126 ng/ml (range 35-320 ng/ml). There is a significant difference between CALGRANULIN C values of patients with acute exacerbation before treatment and after treatment. Mean CALGRANULIN C level in sputum of CF patients with acute exacerbation was 5,600 ± 4,350 ng/ml.

The individual time course of CALGRANULIN C levels in 21 cases of acute exacerbation are shown in *Figure 1*. Not all of the patients reached values inside the normal range, especially when presenting with extremely high levels at the start of antibiotic therapy.

### Inflammatory parameters for comparison

We found CRP elevated in 13 of 21 cases of acute exacerbation before initialisation of antibiotic therapy (61%). There was a significant difference between mean concentrations of CRP in patients with acute exacerbation before (1.87 ± 2.94 mg/dl; range 0-10.6) and after antibiotic therapy (0.15 ± 0.39 mg/dl; range 0-1.6). Nevertheless, mean differences between acute exacerbation and out-patients without acute infection (0.52 ± 0.40 mg/dl; range 0-1.5) were not significant. ESR was above the normal range in 14 of 21 cases (66%). We found a significant difference for mean ESR between patients with acute exacerbation (25 ± 18 mm/h; range 4-51) and out-patients (12 ± 9 mm/h; range 1-28). ESR of patients with acute exacerbation before and after antibiotic therapy (17 ± 15 mm/h; range 6-36) did not differ significantly. In 12 cases (56%), the leukocyte counts were above 10.000/µl. Leukocyte counts were significantly higher in acute exacerbation before (11,260 ± 3,948/µl; range 2,900-22,100) than after antibiotic treatment (7,920 ± 2,311/µl; range 2,500-12,500), but no such difference was found between patients with acute exacerbation before treatment and out-patients (9,583 ± 3,438/µl; range 4,300 - 16,500). Data are summarised in *Figure 2**.*

CALGRANULIN C is therefore potent and reliable as a marker for acute CF-exacerbation. It is an early marker of inflammation and correlates with disease activity. It is superior to conventional indicators of inflammation in differentiating acute and chronic stages of disease. In particular, determination of serum levels of CALGRANULIN C individual profiles are useful to determine states of acute exacerbation.

### Example 2: Identification of CALGRANULIN C as a marker useful in monitoring Kawasaki disease

### Patients and healthy controls

We analysed CALGRANULIN C by use of the ELISA method described above as well as CRP levels of 6 female and 15 male patients (mean age 2.5 years; range 0.4-7.2) fulfilling the criteria of Kawasaki disease, who were treated with intravenous gammaglobulin (2 g/kg body weight). Concentrations of CALGRANULIN C in the serum of Kawasaki patients were determined by a double sandwich enzyme linked immunosorbent assay (ELISA) systems described in *Example 1*. Also, protein and antibody preparation were performed as described above. Serum samples were taken at start of therapy, directly after treatment with gammaglobulin, 2 weeks after start of therapy, and in remission. Mean duration of fever was 7.5 days (range 5-13). The mean maximum of white blood cell count was 14,900/µl (range 5,300-24,400), with an average of 63% neutrophils. 8 patients had coronary artery lesions (CAL) and were diagnosed with coronary aneurysms. All patients with CAL were male. There was no significant difference in age distribution between patients with and without CAL (mean age 2.4 vs. 2.6 years). Patients with CAL had longer duration of fever and higher levels of CALGRANULIN C, CRP, white blood cells, and neutrophil counts.

### Results of CALGRANULIN C analysis

Mean initial CALGRANULIN C level before therapy was 450 ± 348 ng/ml (range 31-1,330 ng/ml). Mean CALGRANULIN C level decreased significantly after gammaglobulin treatment (236 ± 244 ng/ml; range 9-1071; p < 0.05). The CALGRANULIN C levels after 2 weeks were 84 ± 88 ng/ml (range 15-402). CALGRANULIN C levels detected in complete remission were 83 ± 84 ng/ml (range 6-371). Mean initial CRP level was 8.9 ± 3.5 mg/dl (range 2.5 - 16.0 mg/dl). Mean CRP levels decreased to 6.3 ± 6.9 mg/dl (range 0.8-28.7 mg/dl) after gammaglobulin treatment, without showing a significant difference to initial levels. Mean CRP levels were 1.5 ± 2.1 mg/dl (range 0-8.9 mg/dl) after 2 weeks, and 0.15 mg/dl (range 0-0.6 mg/dl) in remission. *Figure 3* shows detected CALGRANULIN C and CRP levels in the course of Kawasaki disease.

Mean CALGRANULIN C in 16 healthy controls (mean age 10.9; range 3-17) was 50 ± 32 ng/ml. Levels higher than two standard deviations above the mean were identified as abnormal, leading to a cut-off value of 115 ng/ml. Two patients had CALGRANULIN C levels within the normal range over the whole course of the disease. These patients had mild disease without coronary aneurysms and fever for only 5 and 6 days, respectively.

Patients with coronary artery aneurysms had higher initial and maximum CALGRANULIN C and CRP levels than patients without cardiac complications, and hence the difference for CALGRANULIN C concentrations was greater than for CRP (*Figure 4*).

The present study indicates that the calcium-binding protein CALGRANULIN C is a potent marker for Kawasaki disease with a sensitivity of 91 %. Serum levels correlated with disease activity in individual patients. CALGRANULIN C is able to determine response to therapy early after gammaglobulin treatment It is the only parameter with highly significant differences between patients with Kawasaki disease before gammaglobulin treatment and after treatment. Furthermore, it is superior to CRP in identifying cases at high risk for coronary artery lesions. Hence, CALGRANULIN C is an early indicator of acute inflammation in the cascade of vasculitis and possibly other autoimmune disorders.

### Example 3: Identification of CALGRANULIN C as a marker useful in the early identification of Systemic onset juvenile arthritis (SOJRA), especially by discrimination from bacterial infection

Using the CALGRANULIN C ELISA described above in detail, we analysed serum concentrations of CALGRANULIN C proteins in patients with SOJRA, in patients with active oligoarthritis form of juvenile rheumatoid arthritis (JRA), in patients with bacterial infections (CRP-value > 50mg/l; average CRP value: 95 mg/l) and in control persons (n=20). In addition, CALGRANULIN C concentrations in the synovial fluid of JRA patients were measured in order to prove the suitability of CALGRANULIN C as local inflammation marker.

Surprisingly it was found, that CALGRANULIN C serum levels were dramatically elevated in SOJRA patients, while they were only moderately elevated both in JRA patients and in patients with bacterial infections (*Figure 5*): CALGRANULIN C concentrations are significantly about 10-fold higher in SOJRA patients compared to JRA patients and to patients with bacterial infections. Hence, CALGRANULIN C is the first marker to reliably discriminate between SOJRA and bacterial infections.

Also, the ratio of CALGRANULIN C concentration and CRP concentration was found to be an excellent and reliable measure for diagnosing SOJRA with high specificity and sensitivity (> 80%).

### Example 4: Identification of CALGRANULIN C as a marker for relapse risk of juvenile rheumatoid arthritis (JRA) patients after first successful treatment

The CALGRANULIN C concentrations in the serum of patients in clinical remissions at the endpoint of the therapy with methotrexat (MTX) were determined. Also CRP and ESR were determined. We compared the values of two groups: Group 1: relapse of the disease within one year. Group 2: no relapse within 1 year, i.e. long-term remission. Surprisingly it was found, that only CALGRANULIN C serum concentrations were significantly different between the two groups and are therefore suitable for the prognosis and therefore for adequate treatment. ESR was found to be not suitable at all. CRP is negative in all patients (n=8) investigated, with the exception of two; hence, sensitivity is highly inadequate.

Therefore, CALGRANULIN C could be identified as the first marker for the determination of the disease activity in JRA patients, especially for diagnosing the relapse risk.

### Example 5: Identification of CALGRANULIN C as a marker for rheumatoid and psoriatic arthritis

### Patients and healthy controls

We investigated 42 patients with chronic inflammatory arthritis. CALGRANULIN C concentrations were analysed in serum and synovial fluid using a sandwich-ELISA as described above. Serum levels were determined from 14 patients (9 male, 5 female; mean age 40 years; range 21-64) suffering from psoriasis arthritis (mean disease duration 14.6 ± 8.6 months), who where treated with the anit-inflammatory drug MTX (mean dose 12.9 ± 4.8 mg). No other medication apart from non-steroidal anti-inflammatory drugs (NSAIDs) were taken. Serum was obtained before and after treatment (mean follow up interval 6.4 ± 1.3 months). In addition, paired serum and synovial fluid samples were available from 28 patients who underwent arthroscopy (8 patients with psoriatic arthritis, 9 patients with rheumatoid arthritis, 11 patients with seronegative arthritis). All patients were examined by the same physician. Clinical status of patients was documented by recording early morning stiffness, pain score, Ritchie articular index (RI) (Ritchie et al., 1968, QJ Med 37: 393-406) and number of swollen joint count (SJC). Patients with rheumatoid arthritis had significantly more affected joints according to SJC and RI than those with seronegative arthritis. Patients with seronegative arthritis were in between these groups. In addition to the clinical status, CRP, ESR, anti nuclear antibodies (ANA), and rheumatoid factor (RF) were documented.

Normal levels of CALGRANULIN C were determined in the serum of 15 healthy adults without signs of inflammation, who either underwent routine blood tests at the University hospital Muenster or volunteered in our laboratories. Patients and controls did not differ in age or gender distribution. Data of patients and healthy controls are summarized in *Table 1*.

**Table 1: Characteristics of patients with psoriatic arthritis (PsA), rheumatoid arthritis (RA) and seronegative arthritis (SA), and healthy controls, respectively.**

| | PsA | PsA (MTX group) | RA | SA | Controls |
|---|---|---|---|---|---|
| Patients (No.) | 8 | 14 | 9 | 11 | 15 |
| Male/Female | 5/3 | 9/5 | 5/4 | 6/5 | 10/5 |

| Age (years) | | | | | |
|---|---|---|---|---|---|
| Mean | 43 | 40 | 53 | 33 | 32 |
| Range | 28-67 | 21-64 | 28-72 | 18-45 | 19-43 |

| Activity (points) | | | | | |
|---|---|---|---|---|---|
| RI | 6.3 ± 2.4 | 6.3 ± 1.5 | 15.7 ± 3.2 * | 2.2 ± 0.6 | n.d. |
| SJC | 9.8 ± 3.7 | 9.6 ± 2.0 | 17.2 ± 3.2* | 1.9 ± 0.6 | n.d. |

| Medication | | | | | |
|---|---|---|---|---|---|
| NSAIDs (No.) | 4 | 14 6 | | 7 | 0 |
| Steroids (No.) | 0 | 0 | 1 | 0 | 0 |

| Laboratory | | | | | |
|---|---|---|---|---|---|
| CRP (mg/dl) | 5.2 ± 2.3 * | 3.6 ± 1.2 | 5.4 ± 1.8 * | 2.3 ± 0.7 | n.d. |
| ESR(nm/h) | 37±12* | 28 ± 9 | 49 ± 8 * | 25 ± 5 | 4 ± 1 |
| ANA⁺ (No.) | 0 | 2 | 5 | 4 | n.d. |

| | | | | | |
|---|---|---|---|---|---|
| RI = Ritchie articular index; SJC = swollen joint count; SF = synovial fluid; n.d. = not determined; Data expressed as mean ± SEM except otherwise stated; * p < 0.05 | | | | | |

### Statistical analysis

Mann-Whitney U test (for unpaired values without normal distribution) and Wilcoxon test (for paired variables) was performed to determine significant differences between distinct categories. SPSS for windows version 9.0 was used to determine correlation of CALGRANULIN C with other parameters. Data are expressed as mean ± SEM. *P* values greater 0.05 were considered to be not significant.

### Results of CALGRANULIN C analysis in serum and synovial fluid

CALGRANULIN C serum levels were highest in rheumatoid arthritis (mean 340 ± 90 ng/ml), and markedly elevated in psoriatic arthitis (mean 260 ± 60 ng/ml), and less but still significantly elevated in seronegative arthritis (190 ± 20 ng/ml) compared to healthy controls (60 ± 20 ng/ml). In paired samples of serum and synovial fluid we found 5 to 10-fold higher CALGRNULIN C levels in synovial fluid than in serum in all patients. Synovial fluid levels of CALGRANULIN C were higher in seronegative arthritis (4,920 ± 1,680 ng/ml) than in rheumatoid arthritis and psoriatic arthritis (1,870 ± 1,160 ng/ml and 1,720 ± 425 ng/ml, respectively). Serum concentrations of CALGRANULIN C correlated well with other parameters used to determine disease activity, most significantly with ESR (r = 0.47; p < 0.01) and RI (r = 0.36; p < 0.01). Data are summarised in *Figure 6*.

Here it is demonstrated that CALGRANULIN C serum levels are a useful marker for local disease activity in different form of arthritis.

### Local expression of CALGRANULIN C in synovial tissue

To confirm local expression of CALGRANULIN C at sites of inflammation we performed immunohistochemical studies. Synovial biopsies were performed in 4 patients with psoriatic arthritis before and after MTX-therapy. In addition, biopsies were obtained from 5 patients with psoriatic arthritis not receiving MTX, 2 patients with rheumatoid arthritis, and 2 patients with seronegative arthritis. Two biopsies of patients without synovial inflammation served as negative controls. Cryo-fixed and paraffin-embedded sections were prepared as commonly known in the art. Rabbit anti-human CALGRANULIN C antibody was used to detect CALGRANULIN C expression. Mouse-anti-human CD15, a granulocyte-associated antigen, was used to detect granulocytes in infiltrates. Mouse-anti human CD163 antibody (clone RM3/1, detecting a macrophage-specific scavenger receptor) was employed to characterize macrophages in infiltrates. Species matching control antibodies of irrelevant specificity were used as negative controls. Finally the sections were counterstained with Mayer's haematoxylin. Secondary antibodies and substrates for colour reactions were used as described before. For double-labelling experiments, anti-CALGRANULIN C antibody was followed by anti-CD15 antibody. We used affinity-purified goat-anti-mouse or goat-anti-rabbit secondary antibodies conjugated with either Texas Red or FITC (Dianova, Hamburg, Germany). Fluorescence was analysed using a Zeiss Axioskop connected to an Axiocam camera supply with Axiovision 3.0 for windows (Zeiss, Göttingen, Germany). No cross-reactivity or spillover was detected in control experiments after omitting specific antibodies or replacing them by isotype-matched control antibodies of irrelevant specificity.

Results are shown in *Figure 7*. No CALGRANULIN C was found in synovial tissue of controls without arthritis. We found expression of CALGRANULIN C in inflamed synovial tissue of patients with rheumatoid arthritis, seronegative arthritis, and psoriatic arthritis. In rheumatoid arthritis and seronegative arthritis, we found CALGRANULIN C-positive cells in infiltrates and the lining layer. There was a diffuse staining for CALGRANULIN C in association with infiltrates, indicating extracellular CALGRANULIN C after secretion by infiltrating granulocytes. There was a distinct expression pattern of CALGRANULIN C in psoriatic arthritis compared to rheumatoid arthritis and seronegative arthritis with a strong association of CALGRANULIN.C expression with small blood vessels. CALGRANULIN C was expressed by granulocytes that adhered to the endothelium of synovial vessels and in perivascular infiltrates. CALGRANULIN C seemed to be released by cells at the endothelium in inflamed synovia of psoriatic arthritis as well. Co-staining with CD15 revealed that mainly granulocytes expressed CALGRANULIN C. We proved co-expression of CALGRANULIN C and CD 15 in double-labelling experiments using immunofluorescence microscopy. Staining for CD 163 clearly revealed a different pattern for macrophages, which contributed to the majority of cells in inflamed synovial tissue.

In this first analysis of human synovial tissue of different forms of arthritis we found a clear difference in the distribution of CALGRANULIN C in psoriatic arthritis compared to rheumatoid arthritis and seronegative arthritis. We found a distinct distribution of CALGRANULIN C with perivascular pronunciation. The perivascular expression pattern in PsA points to a possible role for CALGRANULIN C in angiogenesis associated with this form of arthritis.

### Correlation of CALGRANULIN C with disease activity in response to treatment

We analysed the effects of MTX treatment on CALGRANULIN C expression in serum of 14 patients with psoriatic arthritis and in synovial membranes of 4 patients. Before treatment, extensive expression of CALGRANULIN C was found in synovial tissue of psoriatic arthritis patients before MTX treatment (*cf.* *Figure* 7G), as described above predominantly in the sublining layer and perivascular. CALGRANULIN C expression was almost undetectable in synovial biopsies of the same patients after effective MTX treatment (*cf.* *Figure* 7H). Evaluations are shown in *Table 2.*

**Table 2: Immunohistochemical analysis of CALGRANULILN C expressed in synovial tissue**

| | Before MTX treatment | After MTX treatment |
|---|---|---|
| Patient 1 | 23 | 3 |
| Patient 2 | 12 | <1 |
| Patient 3 | 17 | <1 |
| Patient 4 | 21 | <1 |

| | | |
|---|---|---|
| Synovial membrane was obtained from 4 patients before and after initiation of MTX treatment All sections were evaluated for the number of CALGRANULIN C-positive cells per randomly selected fields at a magnification of 400-fold. At least 10 fields per section were analysed. The mean score of 10 fields was calculated. | | |

All patients improved significantly in clinical scores according to RI, pain score, SJC, and early morning stiffness. CRP and ESR levels also decreased. Response to therapy was paralleled by a marked decrease of CALGRANULIN C serum levels after MTX treatment (mean 240 ng/ml prior versus 100 ng/ml after MTX; *cf.* *Figure* 8). CALGRANULIN C levels correlated well with improving EMS, pain score, RI and SJC. Data are summarised in *Table 3.*

**Table 3: Improvement of disease activity in patients with psoriatic arthritis after initiation of MTX treatment**

| | Before MTX | After MTX |
|---|---|---|
| EMS (min) | 76 | 25 ** |
| Pain (points) | 5.3 | 3.2 * |
| RI (points) | 6.3 | 1.9 ** |
| SJC (No.) | 9.6 | 3.3 ** |
| ESR (mm/h) | 28 | 12 * |
| CRP (mg/dl) | 4.2 | 2.5 * |

| | | |
|---|---|---|
| *p<0.05; **p<0.01 | | |

This study indicates for the first time a role for human CALGRANULIN C in the pathogenesis of synovial inflammation in rheumatoid arthritis, seronegative arthritis, and particularly psoriatic arthritis. Analyses of CALGRANULIN C in synovial fluid and serum indicate that this protein is expressed and secreted at local sites of inflammation in synovitis. Data on CALGRANULIN C in inflammation have been only published for the murine system yet (Hofmann et al., 1999, Cell 97: 889-901; Schmidt et al., 2001, J Clin Invest 108: 949-955). This study is also the first to demonstrate the up-regulation of local CALGRANULIN C expression in synovial tissue resulting in elevated concentrations in serum and synovial fluid of patients with chronic active arthritis. Analyses of synovial tissue of patients with psoriatic arthritis before and after initiation of MTX-therapy revealed a strong correlation of CALGRANULIN C expression with improving disease activity which was reflected by a decrease of CALGRANULIN C serum concentrations.

We furthermore demonstrate that CALGRANULIN C serum levels are a useful marker for local disease activity in different forms of arthritis. Patients with active arthritis revealed significantly higher CALGRANULIN C'levels than healthy controls. We found about 10-fold higher concentrations of CALGRANULIN C in synovial fluid of patients. The high local expression of CALGRANULIN C at the site of inflammation seems to be responsible for the correlating levels that are detected in serum. In this context, the higher levels in synovial fluid of patients with seronegative arthritis in comparison with serum levels correlated with the smaller numbers of affected joints. In psoriatic arthritis and especially rheumatoid arthritis, the greater number of inflamed joints with secretion of CALGRANULIN C is likely to result in the higher concentrations of CALGRANULIN C found in serum. In psoriatic arthritis, CALGRANULIN C levels reflected successful immunosuppressive treatment with MTX. CALGRANULIN C was a reliable marker of the effects of MTX therapy in serum and synovium. The profound effect of MTX on CALGRANULIN C expression in the synovia of psoriatic arthritis patients might be due .to the reduction of proinflammatory cytokines that activate neutrophils and induce CALGRANULIN C expression (Dolhain et al., 1998, Br J Rheumatol 37: 502-508). On the other hand there is a direct effect of MTX on neutrophil chemotaxis that might inhibit the migration of neutrophils into synovial tissue (Kraan et al., 2000, Arthritis Rheum 43: 1488-1495).

The expression of CALGRANULIN C in human arthritis provokes the question whether this protein and its interaction with RAGE might be a target for novel therapies. In different mouse models of inflammation including arthritis, blocking this interaction with soluble RAGE (sRAGE) and anti-CALGRANULIN C antibodies revealed clear anti-inflammatory effects (Hofmann et al., 1999, Cell 97: 889-891; Schmidt et al., 2001, J Clin Invest 108: 949-955). The evidence for a functional role of CALGRANULIN C in human arthritis together with the beneficial effects of blocking agents in mouse models of inflammation make this protein attractive for the development of new biologic therapies that focus on proinflammatory activities of human CALGRANULIN C.

This example thus demonstrates, that serum CALGRANULIN C is suited as a highly sensitive marker which enables monitoring (by measuring) the success of the treatment in rheumatoid arthritis.

### Example 6: Use of CALGRANULIN C as a marker for determining the stage of disease in inflammatory intestinal disease

### Patients and healthy controls

Crohn's disease patients (n=40), ulcerative colitis patients (n=34) and healthy controls (n=30) were investigated: CALGRANULIN C protein serum levels were measured as described above using ELISA. In parallel, CRP and ESR were determined. Disease activity in Crohn's disease was documented by using the Crohn's disease activity index (CDAI; Best et al., 1976, Gastroenterology 70: 439-444), and for ulcerative colitis by using the colitis activity index (CAI; Rachmilewitz, 1989, Br Med J 298: 82-86) and using the criteria of Truelove and Witts (1955, Br Med J 2: 1041-1048). Data of patients are summarised in *Table 4.*

**Table 4: Characteristics of patients with inflammatory intestinal diseases**

| | Crohn's disease | Ulcerative colitis |
|---|---|---|
| Number of patients | 40 | 34 |
| Females/males | 28/12 | 10/24 |

| Age (yrs) | | |
|---|---|---|
| Mean | 32 | 33 |
| Range | 18-56 | 19-60 |

| Disease activity * | | |
|---|---|---|
| Active | 30 | 15 |
| Inactive | 10 | 19 |

| Medication | | |
|---|---|---|
| Steroids | 23 | 21 |
| 5-ASA or sulfasalazine | 36 | 33 |
| Azathioprin | 8 | 8 |
| Infliximab | 3 | 0 |
| Without treatment | 0 | 1 |

| | | |
|---|---|---|
| * *Assessment of disease activity using CDAI in CD and CAI in UC, respectively. Active disease was defined as CDAI > 150 or CAI* =*4.* | | |

In addition, 10 of our patients (6 with Crohn's disease and 4 with ulcerative colitis) were followed up over a period of 8 months (range 3-12) to determine correlation of CALGRANULIN C serum levels with individual courses of disease activity.

Healthy controls were without signs of inflammation (14 male, 16 female; mean age 34 yrs; range 19-57), who either underwent routine blood tests at the Münster University Hospital or volunteered in our laboratory. There were no significant differences for age or gender distribution between controls and patients.

### Statistical analysis

Mann-Withney U-test was performed to determine significant differences of CALGRANULIN C and CRP expression between distinct categories. Correlation of serum markers with disease activity was analysed with Pearson's test using software SPSS version 9.0 for Windows. Data are expressed as mean value ± 95% confidence interval. P values greater 0.05 were considered to be not significant.

### Results of CALGRANULIN C serum analysis

Crohn's disease patients (CDAI > 150, n=30) had significantly elevated levels compared to healthy controls (470 ± 125 ng/ml vs. 75 ± 15 ng/ml; p>0.001). There was also a significant difference between CALGRANULIN C serum levels in patients with active Crohn's disease compared to inactive disease (470 ± 125 ng/ml vs. 215 ± 95 ng/ml; p>0.01). Even patients with inactive disease revealed serum levels that differed significantly from healthy controls (215 ± 95 ng/ml vs. 75 ± 15 ng/ml; p>0.05). Hence, disease activity could be accurately monitored. Moreover, it could be demonstrated that CALGRANULIN C levels strongly correlated with CDAI, supporting superior suitability for diagnosing the stage of disease.

In patients with chronic active ulcerative colitis (CAI ≥4; /n=15), CALORANULIN C levels were also significantly elevated 0 compared to healthy controls (400 ± 120 ng/ml vs. 75 ± 15 ng/ml; p<0.001). The difference between serum levels in active and inactive ulcerative colitis (400 ± 120 ng/ml vs. 115 ± 55 ng/ml; p<0.001) was more pronounced than in Crohn's disease. In contrast to Crohn's disease, patients with inactive ulcerative colitis had serum levels comparable to those of healthy controls. Moreover, it could be demonstrated, that CALGRANULIN C levels strongly correlated with disease activity as determined by Truelove and Witt's index, supporting superior suitability for diagnosing the stage of disease. Thus, CALGRANULIN C is a potent serum maker for the disease stage of chronic inflammatory bowel disease, especially for Crohn's disease and ulcerative colitis.

CRP levels were higher in patients with active Crohn's disease compared to inactive disease (2.0±1.0 ng/ml vs. 0.3±0.3 ng/ml; p<0.05). There was no significant difference between CRP levels of patients with active ulcerative colitis compared to patients with inactive disease (1.1 ± 0.9 mg/dl vs. 0.4±0.3 mg/dl). ESR was significantly higher in patients with active Crohn's disease (22 ± 7 mm/h versus 9 ± 4 mm/h; p<0.01). However, ESR did not differ significantly between groups of ulcerative colitis patients (10 ± 5 mm/h versus 12 ± 5 mm/h). Data are summarised in *Figure 9**.*

We could further demonstrate that CALGRANULIN C serum levels strongly correlated with disease activity in Crohn's disease (r=0.52, n=40, p<0.01) as well as ulcerative colitis (r=0.70, n=34, p<0.001) (*cf. Table 5* below). CRP levels were also higher in patients with active Crohn's disease compared to patients with inactive disease, but at a lower significance level (2.0 mg/dl vs. 0.3 mg/dl ; p<0.05). Interestingly, only in Crohn's disease there was a correlation with CRP and ESR whereas no correlation for these markers with disease activity could be found in ulcerative colitis. The questionable accuracy of these classical markers in inflammatory intestinal disease is in accordance with previous reports (Nielsen et al., 2000, Am J Gastroenterol 95: 359-367; Niederau et al., 1997, Hepatogastroenterology 44: 90-107).

**Table 5: Correlation of serum CALGRANULIN C, CRP and ESR with disease activity in inflammatory intestinal disease**

| | CALGRANULIN C | CRP | ESR |
|---|---|---|---|
| CDAI in CD | r = 0.52 (n=40) | r = 0.44 (n=25) | r = 0.32 (n=28) |
| p | <0.01 | <0.01 | <0.05 |
| CAI in UC | r = 0.70 (n=34) | r = 0.35 (n=26) | r = - 0.1 (n=25) |
| p | < 0.001 | n.s. | n.s. |

| | | | |
|---|---|---|---|
| n.s. = not significant n.s. = not significant | | | |

Individual follow-up data of CALGRANULIN C serum levels in 10 patients with inflammatory bowel disease (6 with Crohn's disease and 4 with ulcerative colitis) over a period of 8 months (range 3-12) showed a strong correlation with disease activity. In patients with ulcerative colitis, individual follow-up data displayed that CALGRANULIN C levels correlated better with disease activity than established markers of inflammation such as ESR (*Figure 10*). CALGRANULIN C serum levels decreased rapidly after treatment with infliximab (*Figure 11*).

### Immunohistochemistryl/Immunofluorescence microscopy

Paraffin-embedded and frozen sections of bowel biopsies from patients with either active Crohn's disease or active ulcerative colitis, and controls without intestinal inflammation were used to detect CALGRANULIN C expression by rabbit anti-CALGRANULIN C antibody. Disease activity was determined in haematoxylin and eosin stained sections. Monoclonal mouse anti human granulocyte-associated antigen CD15 antibody (Dako, Hamburg, Germany), a sensitive neutrophil marker, was used to detect neutrophils in infiltrates. Staining on serial sections was performed to detect co-expression of CALGRANULIN C and CD 15 in infiltrates. For controls, monoclonal mouse IgM (Dianova, Hamburg, Germany) and polyclonal rabbit IgG (Amersham Biosciences, Freiburg, Germany) of irrelevant specificity were employed. Secondary antibodies and substrates for colour reaction were used as described before (Rammes et al., 1997, J Biol Chem 272: 9496-9502; Frosch et al., 2000, Arthritis Rheum 43: 628-637). Immunofluorescence microscopy was carried out as described above for *Example 5.*

Immunohistochemical studies on tissue from patients with inflammatory intestinal disease showed a specific pattern of CALGRANULIN C expression by infiltrating cells in inflamed areas whereas no staining could be found in tissue from patients with inactive disease. In addition, CALGRANULIN C was found in an extracellular distribution surrounding CALGRANULIN C-positive cells, reflecting secretion of CALGRANULIN C and possibly binding to other receptor-bearing cells in infiltrates. In tissue from patients with active Crohn's disease, CALGRANULIN C was detected around granulomatous lesions (*Figure 12* A, B)..In ulcerative colitis, crypt abscesses consisted of a majority of CALGRANULIN C-positive cells (*Figure 12*D). Cells that transmigrated through the epithelium into the lumen also appeared to be CALGRANULIN C-positive in Crohn's disease as well as in ulcerative colitis. Co-staining with monoclonal anti-CD15 provided evidence that expression of CALGRANULIN C was restricted to neutrophils that infiltrated the inflamed tissue (*Figure 12*H).

Taken together, our data demonstrate that CALGRANULIN C is a pro-inflammatory protein that plays a predominant role during inflammatory intestinal disease. It is strongly expressed in inflamed tissue of patients with active Crohn's disease and ulcerative colitis, and circulating levels of CALGRANULIN C seem to be reliable markers of inflammation in monitoring disease activity. Moreover, the beneficial effects of blocking agents in murine models of colitis make CALGRANULIN C an attractive target for novel therapeutic approaches in patients with inflammatory intestinal disease.

### Example 7: CALGRANULIN C is useful as a marker for minimal residual disease activity in juvenile rheumatoid arthritis (JRA) patients after first successful treatment

CALGRANULIN C concentrations in serum were determined for 13 patients with pauciarticular and polyarticular juvenile rheumatoid arthritis who received treatment with MTX to induce remission, and the data were retrospectively investigated for correlation with relapse risk. The CALGRANULIN C concentration was determined at that time when remission was documented according to the JRA criteria. The determination of CALGRANULIN C concentration was performed as described above using an ELISA.

It was found, that 6 patients which were in stable remission for more than 12 months had significantly lower levels when MTX treatment was discontinuated than those 7 patients who had a relapse before 12 months had passed (65 vs. 135 ng/ml CALGRANULIN C; p<0.05; *cf.* *Figure 13*). In contrast, ESR and CRP analysis showed no difference between these patients and were thus not suitable for the prediction of relapse risk. Thus, CALGRANULIN C indicates residual inflammatory disease activity even in the absence of other laboratory or clinical signs of ongoing inflammation. It is thus a predictive marker for stable remission, enabling adequate diagnosis and treatment: patients for which a low risk of relapse is diagnosed do not need to receive MTX which exhibits severe side-effects, while patients with high risk of relapse will be given further MTX treatment as adequate medication.

### Example 8: CALGRANULIN C is secreted by activated neutrophils in vitro

One of the most prominent histological features that is observed in ulcerative colitis as well as in Crohn's disease is the infiltration of neutrophils into the inflamed mucosa at an early time point of inflammation (Nikolaus et al., 1998, Gut 42: 470-476; Kucharzik et al., 2001, Am J Pathos 159: 2001-2009). Recently, it has been shown that CALGRANULIN C is secreted by activated human neutrophils (Boussac & Garin, 2000, Electrophoresis 21: 655-672).

To further prove the relationship between TNF alpha and neutrophil derived CALGRANULIN C, we could demonstrate that TNF alpha was able to stimulate CALGRANULIN C secretion in peripheral neutrophils. Human mixed donor neutrophils were isolated from buffy coats (German red cross, Münster, Germany) as described before (Vogl et al., 1999, J Biol Chem 274: 25291-25296). Briefly, centrifugation through Ficoll-Hypague (Biocoll, Biochrom, Berlin, Germany) was performed to separate neutrophils from mononuclear cells and platelets. Erythrocytes were separated by dextran sedimentation. The remaining cells were washed twice in PBS. Purity of cells was above 95%, as determined by morphological analysis of Trypan-blue stained cells. Neutrophils were resuspended at a final concentration of 1 x 10⁷ cells/ml in serum free RPMI medium (Biochrom, Berlin, Germany) supplemented with 1% glutamine, 1% non-essential amino acids, and 1% penicillin/streptomycin. Secretion was immediately induced by addition of TNF alpha (recombinant human TNF alpha, Cell Biology Boehringer, Mannheim, Germany) to a final concentration of either 2 or 5 ng/ml. Stimulated and non-stimulated cells were incubated for 15 or 30 minutes at 37°C, respectively. Finally, neutrophils were pelleted at 500 x g for 5 minutes at 4°C and the supernatant was saved for analyses of CALGRANULIN C with sandwich-ELISA. Cell lysis was assessed by analysing activity of lactate dehydrogenase (LDH) using its capacity to convert NADH to NAD⁺ and measuring) the decrease of absorbency of NADH at 340 nm. Protease inhibitors were added to prevent proteolytic degradation.

Minimal basal secretion of CALGRANULIN C was determined in unstimulated neutrophils. Concentrations of CALGRANULIN C in the supernatant of cells were between 5 and 10 ng/ml in 3 independent experiments. There was a time- and dose-dependent increase of CALGRANULIN C secretion after stimulation with TNF alpha. There were no differences in viability and cell lysis between our experiments as tested by LDH activity.

The highly significant elevation of the neutrophil derived protein CALGRANULIN C underlines the important role of neutrophils during inflammation such as intestinal inflammation. Neutrophils belong to the very early effector cell population that infiltrate the mucosa and intestinal epithelial cells thereby altering the intestinal barrier function during inflammatory intestinal disease. Elevated circulating levels of serum CALGRANULIN C provide evidence that neutrophils do not only play a role within the local mucosal immune system but are also important in systemic immune responses during chronic active inflammatory intestinal disease.

Here it is also demonstrated that TNF alpha is able to stimulate CALGRANULIN C secretion in peripheral neutrophils. As TNF alpha is hardly detectable in serum and CALGRANULIN C is an extremely stable protein even at room temperature or after multiple thawing and freezing cycles, analysis of serum CALGRANULIN C may provide an excellent marker for the evaluation of response to anti-TNF alpha treatment.
## Claims

1. A method for the diagnosis of inflammatory diseases selected from rheumatoid arthritis, seronegative arthritis, juvenile arthritis, and systemic onset juvenile rheumatoid arthritis (SOJRA), comprising the steps of
a) determining the amount and/or concentration of calgranulin C polypeptide in a serum sample from a patient; and
b) comparing the amount and/or concentration of calgranulin C polypeptide determined in said serum sample with the amount and/or concentration of calgranulin C polypeptide as determined in a serum control sample,
wherein an increase in the amount of calgranulin C polypeptide in the serum sample as compared to the control sample is indicative for said disease.

2. The method according to claim 1, wherein a specific antibody is used for determining the amount and/or concentration of calgranulin C polypeptide.

3. The method according to claim 2, wherein said specific antibody recognises an epitope derived from the amino acid sequence depicted in SEQ ID NO:2.

4. The method according to claims 2 or 3, wherein said antibody is selected from the group comprising polyclonal antiserum, polyclonal antibody, monoclonal antibody, antibody fragments, single chain antibodies and diabodies.

5. The method according to claims 2 to 4, wherein said antibody is used for performing an immunoassay such as an ELISA or an immunohistochemical technique.

6. The method according to any of claims 1 to 5, wherein said disease is juvenile arthritis, and wherein said method further comprises identifying the risk for relapse after first successful treatment.

7. The method according to any of claims 1 to 5, wherein said disease is systemic onset juvenile rheumatoid arthritis (SOJRA), and wherein said method further comprises a discrimination from bacterial infection.

## Patentansprüche

1. Verfahren für die Diagnose von Entzündungskrankheiten, die aus rheumatoider Arthritis, seronegativer Arthritis, juveniler Arthritis und systemisch beginnender juveniler rheumatoider Arthritis (SOJRA) ausgewählt sind, das die folgenden Schritte umfasst:
a) Bestimmen der Menge und/oder Konzentration vom Calgranulin C-Polypeptid in einer Serumprobe von einem Patienten; und
b) Vergleichen der Menge und/oder Konzentration vom Calgranulin C-Polypeptid, die in der genannten Serumprobe bestimmt wurde, mit der Menge und/oder Konzentration vom Calgranulin C-Polypeptid, wie sie in einer Kontrollserumprobe bestimmt wurde,
worin ein Anstieg der Menge vom Calgranulin C-Polypeptid in der Serumprobe im Vergleich zur Kontrollprobe die genannte Krankheit anzeigt.

2. Verfahren nach Anspruch 1, worin ein spezifischer Antikörper zum Bestimmen der Menge und/oder Konzentration vom Calgranulin C-Polypeptid verwendet wird.

3. Verfahren nach Anspruch 2, worin der genannte spezifische Antikörper ein Epitop erkennt, das aus der in der SEQ.-ID Nr. 2 dargestellten Aminosäuresequenz stammt.

4. Verfahren nach Anspruch 2 oder 3, worin der genannte Antikörper aus der Gruppe ausgewählt ist, die Folgende umfasst: polyklonales Antiserum, polyklonalen Antikörper, monoklonalen Antikörper, Antikörperfragmente, Einzelketten-Antikörper und Diabodies.

5. Verfahren nach Anspruch 2 bis 4, worin der genannte Antikörper zur Durchführung eines Immunassays, wie beispielsweise eines ELISA oder einer immunhistochemischen Technik, verwendet wird.

6. Verfahren nach einem der Ansprüche 1 bis 5, worin die genannte Krankheit juvenile Arthritis ist und worin das genannte Verfahren weiter das Identifizieren des Risikos für einen Rückfall nach der ersten erfolgreichen Behandlung umfasst.

7. Verfahren nach einem der Ansprüche 1 bis 5, worin die genannte Krankheit systemisch beginnende juvenile rheumatoide Arthritis (SOJRA) ist und worin das genannte Verfahren weiter eine Unterscheidung von bakterieller Infektion umfasst.

## Revendications

1. Méthode de diagnostic de maladies inflammatoires sélectionnées parmi l'arthrite rhumatoïde, l'arthrite séronégative, l'arthrite juvénile, et l'arthrite rhumatoïde juvénile à apparition systémique (SOJRA), comprenant les étapes consistant à
a) déterminer la quantité et/ou la concentration de polypeptide de calgranuline C dans un échantillon sérique d'un patient ; et
b) comparer la quantité et/ou la concentration de polypeptide de calgranuline C déterminée dans ledit échantillon sérique à la quantité et/ou la concentration de polypeptide de calgranuline C dans un échantillon sérique témoin,
dans laquelle une augmentation de la quantité de polypeptide de calgranuline C dans l'échantillon sérique par rapport à l'échantillon témoin est indicatrice de ladite maladie.

2. Méthode selon la revendication 1, dans laquelle un anticorps spécifique est utilisé pour déterminer la quantité et/ou la concentration de polypeptide de calgranuline C.

3. Méthode selon la revendication 2, dans laquelle ledit anticorps spécifique reconnaît un épitope dérivé de la séquence d'acides aminés représentée dans la SEQ ID N° 2.

4. Méthode selon la revendication 2 ou la revendication 3, dans laquelle ledit anticorps est sélectionné parmi le groupe comprenant un antisérum polyclonal, un anticorps polyclonal, un anticorps monoclonal, des fragments d'anticorps, des anticorps et des diacorps à chaîne unique.

5. Méthode selon les revendications 2 à 4, dans laquelle ledit anticorps est utilisé pour réaliser un dosage immunologique tel qu'une technique ELISA ou immunohistochimique.

6. Méthode selon l'une quelconque des revendications 1 à 5, dans laquelle ladite maladie est l'arthrite juvénile, et dans laquelle ladite méthode comprend en outre l'étape consistant à identifier le risque de rechute après un premier traitement réussi.

7. Méthode selon l'une quelconque des revendications 1 à 5, dans laquelle ladite maladie est l'arthrite rhumatoïde juvénile à apparition systémique (SOJRA), et dans laquelle ladite méthode comprend en outre une discrimination d'une infection bactérienne.
